# EUROPEAN PATENT APPLICATION

(11) **EP 2 392 674 A2**
(43) Date of publication of application: **07.12.2011**
(21) Application number: 11162936.6
(22) Date of filing: 28.04.2006
(51) Int. Cl.: C12Q 1/68, C07H 21/04, G01N 33/50

(54) **Cellular biomarker antioxidant assay and uses thereof**

(30) Priority: 29.04.2005 US 676504 P; 16.02.2006 US 773996 P
(62) Divisional of application: 06751724.3
(71) Applicant: The University of Tennessee Research Foundation, Memphis, TN 38163 (US)
(72) Inventor: Chaum, Edward, Memphis, TN 38163 (US); Lang, John, Fort Worth, TX 76134-2099 (US)
(74) Representative: Van Breda, Jacobus

(57) **Abstract**

The invention encompasses cell-based systems comprising biomarkers that respond to oxidative stress (OS) in a quantitative manner, and methods of use thereof. The systems are useful for screening, identifying and testing antioxidant agents, combinations, and formulations thereof for preventing, treating, or reducing symptoms of conditions associated with oxidative damage to cells. The cell-based systems are useful for identifying effective new antioxidant agents and for optimizing antioxidant formulations for targeted therapeutic applications. One cell-based system utilizes RPE cells of the eye for identifying and optimizing antioxidant compositions effective for treatment of age-related conditions such as macular degeneration. The cell-based systems provide a convenient, inexpensive and physiologically relevant *in vitro* alternative to human population-based methods for testing efficacy of nutritional and pharmaceutical compositions comprising antioxidants. The invention further encompasses nutritional or pharmaceutical compositions targeting particular diseases such as AMD, formulated using methods as disclosed herein.

SEQUENCE LISTING

<110> UNIVERSITY OF TENNESSEE RESEARCH FOUNDATION

<120> CELLULAR BIOMARKER ANTIOXIDANT ASSAY AND USES THEREOF

<130> 1149-004EP

<140> EP 06751724.3
<141> 2007-11-28

<150> PCT/US06/016159
<151> 2006-04-28

<150> 60/773,996
<151> 2006-02-16

<150> 60/676,504
<151> 2005-04-29

<160> 20

<170> PatentIn Ver. 3.3

<210> 1
<211> 19
<212> DNA
<213> Artificial Sequence

<220>
<223> Description of Artificial Sequence: Synthetic
      primer

<400> 1
gtgtgagcgc ttctgcagc                                                19


<210> 2
<211> 19
<212> DNA
<213> Artificial Sequence

<220>
<223> Description of Artificial Sequence: Synthetic
      primer

<400> 2
ccaattcaac ggctcgctt                                                19


<210> 3
<211> 21
<212> DNA
<213> Artificial Sequence

<220>
<223> Description of Artificial Sequence: Synthetic
      primer

<400> 3
ccttccacct cgacgtttac a                                                21

<210> 4
<211> 23
<212> DNA
<213> Artificial Sequence

<220>
<223> Description of Artificial Sequence: Synthetic
      primer

<400> 4
aatcgagtct gtttccagca gaa                                              23

<210> 5
<211> 21
<212> DNA
<213> Artificial Sequence

<220>
<223> Description of Artificial Sequence: Synthetic
      primer

<400> 5
tttcacgtct tggtgccttt t                                                21

<210> 6
<211> 21
<212> DNA
<213> Artificial Sequence

<220>
<223> Description of Artificial Sequence: Synthetic
      primer

<400> 6
ccctcacaat tgcacatgtc a                                                21

<210> 7
<211> 19
<212> DNA
<213> Artificial Sequence

<220>
<223> Description of Artificial Sequence: Synthetic
      primer

<400> 7
gcctatggca tcttcccca                                                   19

<210> 8
<211> 20
<212> DNA
<213> Artificial Sequence

<220>
<223> Description of Artificial Sequence: Synthetic
      primer

<400> 8
ttcgccccct ctgaagttta                                                   20


<210> 9
<211> 20
<212> DNA
<213> Artificial Sequence

<220>
<223> Description of Artificial Sequence: Synthetic
      primer

<400> 9
tgctgcttgt ccaaatcagg                                                   20


<210> 10
<211> 21
<212> DNA
<213> Artificial Sequence

<220>
<223> Description of Artificial Sequence: Synthetic
      primer

<400> 10
cacacatcaa tccccagcag t                                                 21


<210> 11
<211> 19
<212> DNA
<213> Artificial Sequence

<220>
<223> Description of Artificial Sequence: Synthetic
      primer

<400> 11
aaggtccatg aaccctgcc                                                    19


<210> 12
<211> 20
<212> DNA
<213> Artificial Sequence

<220>
<223> Description of Artificial Sequence: Synthetic
      primer

<400> 12
ggcactggaa cctgctgaaa                                                    20


<210> 13
<211> 20
<212> DNA
<213> Artificial Sequence

<220>
<223> Description of Artificial Sequence: Synthetic
      primer

<400> 13
caggtgcggg cattctatgt                                                    20


<210> 14
<211> 20
<212> DNA
<213> Artificial Sequence

<220>
<223> Description of Artificial Sequence: Synthetic
      primer

<400> 14
ccggcaatgt tctcacacag                                                    20


<210> 15
<211> 21
<212> DNA
<213> Artificial Sequence

<220>
<223> Description of Artificial Sequence: Synthetic
      primer

<400> 15
tttcacgtct tggtgccttt t                                                  21


<210> 16
<211> 21
<212> DNA
<213> Artificial Sequence

<220>
<223> Description of Artificial Sequence: Synthetic
      primer

<400> 16
ccctcacaat tgcacatgtc a                                                  21

<210> 17
<211> 21
<212> DNA
<213> Artificial Sequence

<220>
<223> Description of Artificial Sequence: Synthetic
      primer

<400> 17
ccacccttga gaatccttcc t                                                21


<210> 18
<211> 21
<212> DNA
<213> Artificial Sequence

<220>
<223> Description of Artificial Sequence: Synthetic
      primer

<400> 18
ggagccacag agcatgagat g                                                21


<210> 19
<211> 20
<212> DNA
<213> Artificial Sequence

<220>
<223> Description of Artificial Sequence: Synthetic
      primer

<400> 19
caccctgaag taccccatcg                                                  20


<210> 20
<211> 21
<212> DNA
<213> Artificial Sequence

<220>
<223> Description of Artificial Sequence: Synthetic
      primer

<400> 20
tgccagattt tctccatgtc g                                                21

## Description

### CROSS-REFERENCE TO RELATED APPLICATIONS

The present application claims priority under 35 U.S.C. §119(e) to U.S. Provisional Application Nos. 60/676,504, filed April 29, 2005; and 60/773,996, filed February 16, 2006, both entitled "Cellular Biomarker Antioxidant Assay and Uses Thereof," the entirety of each of which is hereby incorporated by reference.

### FIELD OF THE INVENTION

The invention generally relates to cell-based systems and methods for assaying, screening and identifying effective and appropriate antioxidant agents or combinations thereof. Such agents are useful for preventing, treating, or reducing symptoms of a wide variety of disorders associated with oxidative damage to cells. More particularly, the systems provide cell type-appropriate methods for optimizing antioxidant formulations for targeted therapeutic applications, such as treating age-related degenerative conditions.

### BACKGROUND

A very wide range of disorders affecting all major systems in the body involve damage caused to tissues and cells by oxidative stress, a condition wherein cells and tissue are subjected to exposure to damaging reactive oxygen species including free radicals such as the hydroxyl radical, peroxide, singlet oxygen, superoxide, and their reactive byproducts. Oxidative stress can result in accumulation of these destructive molecules and their reactive byproducts, which can alter and destroy cell membranes, proteins or genetic material of cells by "oxidizing" them, and can render them dysfunctional and in some cases destructive.

Antioxidant therapy is widely used in an attempt to counter the devastating effects on cells of reactive oxygen species. Numerous antioxidant agents have been identified and tested for efficacy in reducing cellular oxidative stress in a wide variety of conditions.

In the eye, several serious disorders are thought to be caused or exacerbated by oxidative stress. Age-related macular degeneration (AMD) is a potentially blinding condition that affects about 10 million older Americans, and this number is expected to double in the near future. Up to 90% of AMD sufferers have the so-called "dry" form of the disease, for which there is no effective treatment or cure. One symptom of the "dry" form of the disease is the accumulation of metabolites in the form of dysfunctional proteins and lipids that the cells are unable to remove, ultimately threatening their viability.

Results from epidemiological studies of normal patient populations and those with AMD have indicated that some patients at higher risk of developing AMD are deficient in certain micronutrients such as β-carotene, lutein, and zinc, or may benefit by increasing levels above their basal levels. Subsequent to these observations, a series of prospective clinical trials has been and continues to be conducted, either alone or jointly by the National Institutes of Health and private industry, the most important of these known as the Age-Related Eye Disease Study (AREDS). Based in part on clinical observations, laboratory investigation, and compromise, a combination of antioxidant agents and minerals (including vitamin A as β-carotene, vitamins E and C, zinc, and copper) was tested for its efficacy in reducing the symptoms in patients with AMD. The results showed an effect for the most rapidly changing and most severely afflicted AMD patients (i.e., patients in Categories 3 and 4, comprising approximately 20% of the AMD patients participating in the study) over a period of 5-7 years. Those who did benefit experienced a modest reduction in the rate of progression (about 20-25%) of the disease. No benefit was observed for Category 1 and 2 AMD patients in the AREDS trial because the rate of progression to advanced AMD is slower.

What is needed is a method for identifying novel antioxidants, or combinations of antioxidants, that would benefit a significant fraction of AMD patients. Also needed are methods for determining the combinations of antioxidants that would most benefit each category of AMD patient, respectively. See reports related to the AREDS clinical trials listed in the References section, *infra.*

Imminently, an updated AREDS trial will be underway in an attempt to improve the patient outcome by adding other components such as the xanthophylls lutein and zeaxanthin, omega-three fatty acids (e.g., DHA and EPA), and by optimizing the amounts of the vitamins and minerals found to be effective during the first ARED Study.

Unfortunately, studies such as the AREDS trials are time-consuming, very expensive and provide no guarantee of success or improved results. To improve the success rate of prospective studies of antioxidant agents and formulations, a clear need exists to develop ways to optimize testing of proposed agents and formulations for reduction of oxidative stress in a reproducible, physiologically relevant manner in advance of testing them in patients.

### SUMMARY OF THE INVENTION

It has been discovered that an exemplary cell type important for conditions involving oxidative damage (i.e., the retinal pigment epithelial (RPE) cell of the eye, the target of oxidative damage in age-related macular degeneration) demonstrates a reproducible and quantifiable molecular response to oxidative stress (OS). When subjected to oxidative stress, the cell up- or down-regulates the expression of specific cellular markers involved in the response of the cell to stress ("biomarkers of OS"). The relative level of stress experienced by the cell can be monitored by analyzing expression of one or more of these biomarkers of OS under specific conditions *in vitro.*

Based on these observations, a cell-based assay system and method has been developed that is useful for measuring OS levels in a cell in a reproducible and quantitative fashion. The system has a wide variety of useful applications, including identifying new antioxidant agents, and developing formulations of antioxidant compositions optimized to reduce oxidative stress in a cell. Preferred embodiments of the cell-based system employ specific cell types for discovery of antioxidant compositions and formulations having particular efficacy for conditions based on oxidative damage to specific cell types. A particularly preferred assay utilizes the RPE cells of the eye, which are a target of oxidative damage and the focus of consequent pathology in age-related macular degeneration.

Using the cell-based systems and methods of the invention, it is possible to identify and test antioxidant formulations, minerals, nutritional and pharmaceutical formulations at physiologic levels, and to determine their efficacy in reducing the level of oxidative stress in any cell type or tissue of interest. Based upon proven efficacy in the target tissue *in vitro*, the cell-based assays are a useful proxy for, or adjunct to, human population-based clinical trials as predictors of potential therapeutic efficacy of drug formulations.

Accordingly, one important aspect of the invention is a method for measuring oxidative stress in a cell and, more particularly, the cellular response to that stress. The method includes providing isolated cell populations maintained in culture. Each population comprises a cell type that expresses at least one biomarker of OS that responds to OS by changing its expression level in a quantitative manner. The cell populations are maintained under conditions in which the expression levels of the biomarkers of OS remain unchanged in absence of an inducer of OS. For measurement of OS in the cells, an inducer of OS is provided to a test group and not to a control group of the cell populations. The level of expression of the biomarker of OS is determined in the test and control groups, and a change in the expression level of the biomarker in the test group is correlated with the level of oxidative stress in the cell.

Preferred biomarkers of OS respond to oxidative stress in a reproducible, quantitative manner. Useful molecular markers of OS include one or more of *FosB, JunB, cFos, Fos L, ATF3, CRYBA, TXN,* heme oxygenase *(HO-1), EGR-1,* C1 inhibitor, *AP-1, IGFBP-3, IGFBP-5, IGFBP-6, PLAGL1 (ZAC1*/*LOT1), TIEG, P311,* metallothionein 1X, metallothionein 1L, metallothionein 1H, metallothionein 1H-like, metallothionein 1G, metallothionein 2A, *ETR 101,* thioredoxin δ3, *HSP A1A, HSP A1B, HSP-27,* interleukin 8, *M-GST3, GSTA4, MMP2, DTR, HOS-1,* and *LEDGF.*

Particularly preferred biomarkers of OS are those in which the level of expression changes in response to the level of OS in a dose-dependent manner. Preferred biomarkers of OS that demonstrate dose-dependent responses to inducers of OS include the genes *FosB, Jun B, cFos, ATF3,* and *CRYBA.*

Measurement of expression levels of the molecular markers of OS in the cell can be that of mRNA or protein. A preferred means of measuring levels of mRNA expression is by using the polymerase chain reaction (PCR).

Certain embodiments of the cell-based system of the invention are particularly suitable for assay of antioxidants of potential use in the retina. Accordingly, these systems utilize cell populations that comprise at least one cell type of the retina. In a particularly preferred embodiment, the cell type is a retinal pigmented epithelial (RPE) cell.

Another aspect of the invention is a method for identifying or testing an antioxidant agent. The method comprises providing isolated cell populations, each comprising a cell type that expresses at least one marker that responds to oxidative stress by changing its expression level in a quantitative manner. The cell populations are maintained under conditions in which the expression level of the marker is unchanged in the absence of an inducer of oxidative stress. In the inventive method, oxidative stress is created by adding an inducer of oxidative stress to a first test group of the cell populations. An antioxidant agent is added to a portion of the first test group, to produce a second test group comprising the inducer of OS and the added antioxidant agent. The level of expression of at least one marker of oxidative stress in the cells of the first and second test groups is determined. A quantitative change in expression level of the marker between the first and second groups indicates that the agent is an effective antioxidant.

One non-limiting variation of the method for identifying or testing an antioxidant agent is performed in a multi-well format (such as a 96-well plate) and is useful for testing multiple antioxidant agents in combination, and in a variety of concentrations. The latter method is particularly suited to screening of candidate antioxidant agents and formulations in a high-throughput setting.

In further aspect, the invention provides antioxidant agents identified according to the above-described methods.

Yet another aspect of the invention is a nutritional or pharmaceutical composition comprising pharmaceutical antioxidants, nutritional antioxidants, or a combination thereof, optimized to reduce oxidative stress or damage in a cell type of choice. The nutritional or pharmaceutical composition includes single or multiple classes of antioxidants such as water-soluble vitamin antioxidants; mineral cofactors of antioxidant enzymes; factors that increase the biosynthesis of antioxidant enzymes such as the B vitamins including biotin and folic acid, vitamin C, zinc, copper, manganese, and selenium; oil soluble antioxidants such as carotenoids including pro-vitamin A homologues such as β-carotene, retinoids, and the xanthophylls lutein and zeaxanthin; interfacially active antioxidants such as vitamin E, other tocopherols and tocotrienols; water- and oil-soluble polyphenols including flavones; herb- and plant-derived antioxidants such as carnosol and carnosic acid, organosulfur compounds like allylcysteine, alliin and allicin, and fatty acid forms such as lipoic acid; fatty acid antioxidants with both conjugated and unconjugated unsaturated chains including omega-3 fatty acids, such as EPA (eicosapentaeneoic acid) and DHA (docosahexaeneoic acid); antioxidant amino acids and polypeptides from either essential acids like tryptophan and arginine or their derivatives or plant or tissue extracts such as oyster extracts; isothiocyanates known to be inducers of antioxidant enzymes such glutathione transferase; quinones such as ubiquinols and quinone derivatives such as alkyl quinones, the coenzymes-Q, such as CoQ10; and multifunctional antioxidants such as plant extracts known to regulate nitric oxide, including pycnogenol.

Particularly preferred embodiments of the nutritional or pharmaceutical compositions are based on the response of OS-related biomarkers of ocular cells, such as RPE cells, and are optimized to reduce OS in RPE cells, a prime target of oxidative damage in aging disorders of the eye such as age-related macular degeneration.

Other aspects and advantages of the invention are discussed below.

### BRIEF DESCRIPTION OF THE DRAWINGS

Figure 1 is a graph showing the effect of tissue culture conditions on *FosB* gene expression in a cell-based assay according to an embodiment of the invention.
Figure 2 is a graph showing quantification of transcription of cellular biomarker *FosB* in RPE cells exposed to varying concentrations of H₂O₂, an inducer of oxidative stress (OS), according to an embodiment of the invention.
Figure 3 is a graph showing the effect of tissue culture conditions on gene expression of biomarker *JunB* in a cell-based assay according to an embodiment of the invention.
Figure 4 is a graph showing quantification of transcription of biomarker *FosB* in cultured RPE cells after addition of varying concentrations of an inducer of OS.
Figure 5 is a graph showing quantification of transcription of biomarker *c-Fos* in cultured RPE cells after addition of varying concentrations of an inducer of OS.
Figure 6 is a graph showing quantification of transcription of biomarker *FosL* in cultured RPE cells after addition of varying concentrations of an inducer of OS.
Figure 7 is a graph showing quantification of transcription of biomarker *JunB* in cultured RPE cells after addition of varying concentrations of an inducer of OS.
Figure 8 is a graph showing quantification of transcription of biomarker *cJun* in cultured RPE cells after addition of varying concentrations of an inducer of OS.
Figure 9 is a graph showing quantification of transcription of biomarker *ATF2* in cultured RPE cells after addition of varying concentrations of an inducer of OS.
Figure 10 is a graph showing quantification of transcription of biomarker *ATF3* in cultured RPE cells after addition of varying concentrations of an inducer of OS.
Figure 11 is a graph showing quantification of transcription of biomarker *HO-1* in cultured RPE cells after addition of varying concentrations of an inducer of OS.
Figure 12 is a graph showing quantification of transcription of biomarker *CRYBA* in cultured RPE cells after addition of varying concentrations of an inducer of OS.
Figure 13 is a graph showing quantification of transcription of redox gene biomarkers *CAT, SOD2* and *GSS* in cultured RPE cells after addition of varying concentrations of an inducer of OS.
Figures 14A-B are two schematic diagrams showing a screening matrix for testing antioxidant molecules and compositions, according to an embodiment of the invention.
Figure 15 is a graph showing quantitative assay of OS-related biomarker *HO-1* in stressed human RPE cells treated with an antioxidant agent, according to an embodiment of the invention.
Figure 16 is graph showing quantitative assay of OS-related biomarker *FosB* in stressed human RPE cells treated with an antioxidant agent, according to an embodiment of the invention.
Figure 17 is a graph showing quantitative assay of OS-related biomarker *JunB* in stressed human RPE cells treated with an antioxidant agent, according to an embodiment of the invention.
Figure 18 is a graph showing quantitative assay of OS-related biomarker *ATF3* in stressed human RPE cells treated with an antioxidant agent, according to an embodiment of the invention.
Figure 19 is a graph showing quantitative assay of expression of a control gene, β-actin, in stressed human RPE cells treated with an antioxidant agent, according to an embodiment of the invention.
Figure 20 is a graph showing quantitative assay of OS-related biomarker *CRYBA1* in stressed human RPE cells treated with an antioxidant agent, according to an embodiment of the invention.
Figure 21 is a graph showing quantitative assay of OS-related biomarker *cFos* in stressed human RPE cells treated with an antioxidant agent, according to an embodiment of the invention.
Figure 22 is a graph showing quantitative assay of inhibition of OS-induced transcription of biomarker *FosB* in human RPE cells treated with an antioxidant agent, according to an embodiment of the invention.
Figure 23 is a graph showing quantitative assay of inhibition of OS-induced transcription of biomarker *cFos* in human RPE cells treated with an antioxidant agent, according to an embodiment of the invention.
Figure 24 is a graph showing quantitative assay of inhibition of OS-induced transcription of biomarker *CRYBA* in human RPE cells treated with an antioxidant agent, according to an embodiment of the invention.
Figure 25 is a graph showing quantitative assay of OS-induced transcription of biomarker *ATF3* in human RPE cells, according to an embodiment of the invention. Treatment with Vitamin C does not inhibit OS-induced transcription of this biomarker in these cells.

### DETAILED DESCRIPTION OF THE INVENTION

As discussed, large human populations are currently used in clinical trials to test antioxidant agents and formulations for possible efficacy in disorders involving damage caused by reactive oxygen species. This approach is costly and time-consuming, and cannot provide information as to the biological or biochemical basis for any beneficial response, either by an individual or a population.

An important aspect of the invention is a cell-based system for identifying and testing antioxidant agents and formulations that can reproducibly and quantitatively assess the physiological response of a cell to oxidative stress, and, importantly, the reduction in stress experienced by the cell in the presence of an antioxidant agent. Furthermore, the system provides a means of developing antioxidant agents and formulations that are optimized to reduce oxidative damage in specific cell types that are the target of oxidative damage in particular disorders associated with oxidative stress.

### Oxidative Stress (OS), OS-Related Disorders, and Antioxidants

As used herein, the term "disorder" refers to an ailment, disease, illness, clinical condition, or pathological condition. Particular disorders involving oxidative stress are described *infra.*

As used herein, the term "antioxidant" refers to compounds that neutralize the activity of reactive oxygen species or inhibit the cellular damage done by the reactive species or their reactive byproducts or metabolites. The terms "reactive oxygen species," or "oxidative species," as used herein, refer to oxygen derivatives from oxygen metabolism or the transfer of electrons, resulting in the formation of "free radicals" (e.g., superoxides or hydroxyl radicals).

"Oxidative stress," as used herein, refers to a state of a cell or tissue of an animal; *in vitro* or *in vivo* (or a process by which this state is achieved) whereby a cell or tissue is subj ected to oxidative species that can cause cell damage and disease. For example, oxidative stress can involve accumulation of destructive molecules such as free radicals that damage components of the cell including cell membranes, proteins or genetic material by "oxidizing" them.

An "inducer of oxidative stress," as the term is used herein, refers to any molecule, compound, composition, or more generally a physical or chemical condition that causes a cell to experience oxidative stress. One example of an inducer of oxidative stress is H₂O₂. Other peroxides such as lipid peroxides, superoxide, hydroxyl free radical, photoreactive lipids and proteins are all known to contribute to oxidative stress.

An "antioxidant agent," as used herein, refers to any molecule, compound, composition, formulation, nutritional factor or supplement, or treatment that assists in the prevention or treatment of disorders, or complications of disorders, caused by inducers of oxidative stress such as reactive oxygen species. Antioxidant agents typically act by inhibiting oxidation of cellular components. Such compounds include, by way of example and without limitation, Vitamin E, a fat-soluble, naturally occurring vitamin, which has particularly good anti-oxidant properties, as well as its derivatives, and Vitamin C, as well as its derivatives. Ascorbyl palmitate has been shown to be an effective derivative of vitamin C in some cases. Vitamin A, for example as β-carotene, is also known as an antioxidant. Antioxidant compositions and formulations can further comprise minerals such as copper and zinc, and other components such as xanthophylls (e.g., lutein, zeaxanthin) and omega-three fatty acids (e.g., docosahexaenoic acid, (DHA) and eicosapentaenoic acid (EPA)).

In many circumstances combinations of antioxidants may prove more beneficial than a single antioxidant because a combination of antioxidants can potentiate a full cascade of redox reactions, ultimately reducing the oxidative stress to water or an equivalent ground state. Nutritional and/or pharmaceutical compositions in accordance with the invention can comprise single or multiple classes of antioxidants including but not limited to: water-soluble vitamin antioxidants and mineral cofactors of antioxidant enzymes or factors that increase their biosynthesis such as the B vitamins including biotin and folic acid, vitamin C, zinc, copper, manganese, selenium; oil soluble antioxidants such as carotenoids including especially pro-vitamin A homologues such as beta carotene, retinoids, the xanthophylls lutein and zeaxanthin; interfacially active antioxidants such as vitamin E, other tocopherols and tocotrienols; water- and oil-soluble polyphenols including flavones, isoflavones, flavanones, flavonols, catechins, ginkgolides, anthocyanidins, and proanthocyanidins, and their oligomers and functionalized derivatives, especially glycosidic, ether, and fatty acid derivatives; herb- and plant-derived antioxidants such as carnosol and carnosic acid, organosulfur compounds like allylcysteine, alliin and allicin, and fatty acid forms like lipoic acid; fatty acid antioxidants with both conjugated and unconjugated unsaturated chains such as omega-3 fatty acids, especially EPA (eicosapentaeneoic acid) and DHA (docosahexaeneoic acid); antioxidant amino acids and polypeptides from either essential acids like tryptophan and arginine or their derivatives, or plant or tissue extracts such as oyster extracts; isothiocyanates, like plant- or fruit-derived benzyl isothiocyanate, known to be inducers of antioxidant enzymes such glutathione transferase; quinones such as ubiquinols and quinone derivatives such as alkyl quinones, the coenzymes-Q, such as CoQ10; and multifunctional antioxidants such as plant extracts such as pycnogenol, known to regulate nitric oxide.

Examples of clinical conditions and settings in which oxidative species have been implicated (also referred to herein by the term "oxidative stress (OS)-related disorders," and like terms) include, but are not limited to: smoking, ischemia-reperfusion injury (e.g., stroke/myocardial infarction and organ transplantation); cancer; diabetes; aging; arthritis associated with age; fatigue associated with age; alcoholism; red blood cell defects (e.g., favism, malaria, sickle cell anemia, Fanconi's anemia, and protoporphyrin photo-oxidation); iron overload (e.g., nutritional deficiencies, Kwashiorkor, thalassemia, dietary iron overload, idiopathic hemochromatosis); kidney disorders (e.g., metal ion-mediated nephrotoxicity, aminoglycoside nephrotoxicity, and autoimmune nephrotic syndromes); gastrointestinal disorders (e.g., oral iron poisoning, endotoxin liver injury, free fatty acid-induced pancreatitis, nonsteroidal antiinflammatory drug-induced gastrointestinal tract lesions, and diabetogenic actions of alloxan); inflammatory-immune injury (e.g., rheumatoid arthritis, glomerulonephritis, autoimmune diseases, vasculitis, and hepatitis B virus); nervous system disorders (e.g., Parkinson's disease, amyotrophic lateral sclerosis, neurotoxic disorders, allergic encephalomyelitis, potentiation of traumatic injury, hypertensive cerebrovascular injury, and vitamin E deficiency); disorders of the heart and cardiovascular system (e.g., atherosclerosis, adriamycin cardiotoxicity, Keshan disease (selenium deficiency) and alcohol cardiomyopathy); disorders of the eye (e.g., ocular inflammation or allergy, photic retinopathy, retinal detachment, diabetic retinopathy, cystoid macular edema, ocular hemorrhage, including retinal and choroidal neovascularization and hemorrhage, cataractogenesis, retinitis pigmentosa, and degenerative retinal conditions including congenital and age-related forms of macular degeneration and vascular obstruction or occlusion).

### Cell-Based Assay Systems for Measurement of Oxidative Stress

The disclosed cell-based systems, and methods of use thereof, are based on quantitative measurement of physiological responses to oxidative stress (OS) by one or more cell types. The cell types may be selected as appropriate according to need, for example, for development of an optimized antioxidant therapy for a particular disorder. Unlike an entire human being or animal subject receiving a drug or compound, the inventive cell-based systems can provide target cell- or target tissue-specific information. As shown in Examples *infra,* myriad factors which are subject to variability can be determined by "interrogating" cells, including: the identity of specific gene sets that are affected by oxidative stress in a particular cell or tissue type; quantitative aspects of the response of a particular cell type(s) to oxidative stress at the level of gene or protein expression; and, very importantly for the development of new antioxidant therapies, the level of reduction of oxidative stress experienced by a particular cell type under defined conditions in the presence of, or following treatment with, a test antioxidant agent or formulation.

A fundamental aspect of the invention is the monitoring of particular gene transcripts or gene products which serve as quantitative molecular indicators ("markers," or "biomarkers") of the level of oxidative stress experienced by a cell under a particular set of conditions. As used herein, the terms "marker/biomarker of oxidative stress," "molecular marker/biomarker of stress," "cellular marker/biomarker of oxidative stress" and the like, refer to any nucleic acid or protein molecule or effective fragment or metabolite or stimulated second messenger thereof that responds to oxidative stress experienced by a cell in a reproducible, quantitative manner. In the practice of the invention, the cell-based assay systems and methods utilize isolated cell populations that express markers of oxidative stress. Preferably the biomarkers respond to oxidative stress by changing expression level in a reproducible, quantitative manner. An important criterion for inclusion as a "marker/biomarker of oxidative stress" is an OS-specific response in gene expression, i.e., the maintenance of an unchanged level of expression under normal (control) conditions *in vitro,* in absence of an inducer of oxidative stress.

To measure the response of a cell to oxidative stress, cells are cultured under conditions suitable for assays of OS. As further described in Examples below, great care must be taken to ensure that such conditions are met for the particular cell type and assay methodology in use. As shown, failure to establish and test appropriate culture reagents, conditions and methods of handling cells and reagents can result in erroneous conclusions regarding OS-induced gene expression. For example, certain methods of rinsing cultured cells, and aspirating and replacing media before RNA extraction can significantly affect expression of OS-related gene transcripts in absence of any addition of an exogenous inducer of OS. Accordingly, appropriate conditions must be established, and cellular markers of OS selected for analysis must be those that respond specifically to an inducer of OS. Preferred biomarkers respond to OS in a quantitative, dose-dependent manner, as illustrated in the Examples, *infra.*

In the practice of the cell-based assay, the cells are divided into control and test populations. An inducer of oxidative stress is added to the test populations of cells but not to the control groups, and at selected intervals after addition of the OS inducer, the level of expression of at least one marker of oxidative stress is measured and compared in the test and control populations. A difference in the level of expression of the marker in the presence and absence of the inducer of OS provides a quantitative measure of the level of OS experienced by the cell under the particular experimental conditions.

As discussed, biomarkers of OS suitable for use in a cell-based system in accordance with the invention will depend upon the cell type(s) used in the system, and can be determined experimentally. It is first necessary to select a cell type of interest, for determination of the marker genes, and study of their gene expression in the cell under various control and test conditions.

As an example, to determine the response of a retinal cell to oxidative stress, and to test the therapeutic potential of candidate antioxidant agents on a cell type of the retina, a suitable assay system can assess, for example, cellular stress in RPE cells of the eye. The cell population in the assay can include any suitable population of isolated RPE cells. In some applications, an immortalized RPE cell line, as are known in the art, is preferred for ease of handling and culturing. For determining cellular responses to OS in disease states, the assays may use isolated populations of RPE cells derived from patients (for example during retinal surgery), or from donor eyes of patients known to have suffered during life from a particular eye disorder such as age-related macular degeneration.

One preferred assay using RPE cells utilizes one or more stress-related biomarkers selected *from FosB, JunB, cFos, Fos L, ATF3, CRYBA, TXN,* heme oxygenase *(HO-1), EGR-1,* C1 inhibitor, *AP-1, IGFBP-3, IGFBP-5, IGFBP-6, PLAGL1 (ZAC1lLOT1), TIEG, P311,* metallothionein 1X, metallothionein 1L, metallothionein 1H, metallothionein 1H-like, metallothionein 1G, metallothionein 2A, ETR 101, *thioredoxin δ3, HSP A1A, HSP A1B, HSP-27, interleukin 8, M-GST3, GSTA4, MMP2, DTR, HOS-1, and LEDGF.*

Particularly preferred among these biomarkers are members of the *Fos* and Jun families. As key components of the AP-1 transcription factor complex, these genes are known play a major role in directing the cellular responses to extracellular signaling by inducing diverse patterns of gene expression (Karin & Shaulian). Transcription of these genes is rapidly induced in the absence of new protein synthesis (hence they are termed "immediate early genes, IEG"). The IEG are known to activate the transcription of genes controlling cell proliferation, and commitment to, or protection from, apoptosis through interactions with binding sites in upstream regulatory elements. These genes are activated by several well-described signaling cascades, including the mitogen-activated protein kinases, the Janus kinase/signal transducer and activator of transcription (Jak/STAT) cascade, nuclear factor kappa-B, and others.

Extracellular signaling leads to activation of transcription factors via phosphorylation. The Fos and Jun family proteins (together with ATF) bind as homodimers and/or heterodimers, to form various AP-1 transcription factor complexes. The relative ratio of these homo- and heterodimer complexes is believed to induce different biological responses and activate distinct biological programs within cells. Expression of the IEGs themselves is controlled through transcription factor binding to upstream regulatory elements. Each AP-1 family gene has both common and unique regulatory loci, some of which are specific for a particular signaling pathway.

Other embodiments of the invention utilize cultures comprising other cell types of the retina such as photoreceptors, or mixed cultures, for example comprising cell types of the choroid, optic nerve, trabecular meshwork, cornea, lens, etc. Those of skill in the art will recognize that these are merely examples and that any other suitable cell type from any tissue or source in the body can be used.

To identify suitable biomarkers of oxidative stress for use in a particular cell type, marker genes can be identified in the cell type using any of a number of well-known techniques in the art of molecular biology useful for detection of gene transcripts that are differentially expressed in particular cellular states. Such methods include but are not limited to: differential display, Serial Analysis of Gene Expression (SAGE), subtractive cDNAcloning, screening of gene chips, etc. Example 8 below describes a method of screening a gene array with probes made from cultured RPE cells in the presence and absence of an inducer of oxidative stress, to identify OS-induced molecular markers in this cell type.

See, generally, treatises such as Sambrook et al. in Molecular Cloning: A Laboratory Manual (2d ed. 1989); and Ausubel et al., Current Protocols in Molecular Biology, John Wiley & Sons, New York, 1989, for general disclosure relating to recognized immunological and molecular assays that can be used to detect cell markers, once identified by methods described above.

### Methods and Systems for Identifying and Testing Antioxidant Agents

Another important aspect of the invention is a method for identifying or testing an antioxidant agent. The methods utilize cell-based assays in accord with the invention to determine the level of oxidative stress experienced by a cell under various conditions as a means to test candidate antioxidant compounds for their efficacy in reducing oxidative stress in the cell. Efficacy of a candidate compound or formulation *in vitro* can be shown, for example, by demonstrating reduced indices of stress in a cell subjected to OS in the presence of the compound, relative to that of a control cell subjected to OS alone. Efficacy is assessed on the basis of reproducible, quantitative changes in the responses of specific molecular markers of oxidative stress in suitable cell types.

The methods of identifying and testing antioxidant agents in accord with the invention include the following general steps:
(a) providing isolated cell populations, each comprising a cell type that expresses at least one marker that responds to oxidative stress by changing its expression level in a quantitative manner,
(b) maintaining the cell populations under conditions in which the expression level of the marker is unchanged in the absence of an inducer of oxidative stress;
(c) adding an inducer of oxidative stress to the cell populations of step (b), to form a first test group;
(d) adding an antioxidant agent to a portion of the first test group of step (c), to form a second test group comprising the antioxidant agent; and
(e) determining the level of expression of at least one marker of oxidative stress in the cells of the first and second test groups, wherein a quantitative difference in expression level of said marker between said first and second test groups indicates that the agent is an effective antioxidant agent capable of providing prophylaxis. The antioxidant agent can be added before, during, or after exposure to the oxidative stress.

In the practice of the method, cell populations comprising any suitable cell type or combination of cell types are selected, as discussed above. Typically he cells are plated in tissue culture wells and grown to confluence prior to performing assays involving oxidative stress.

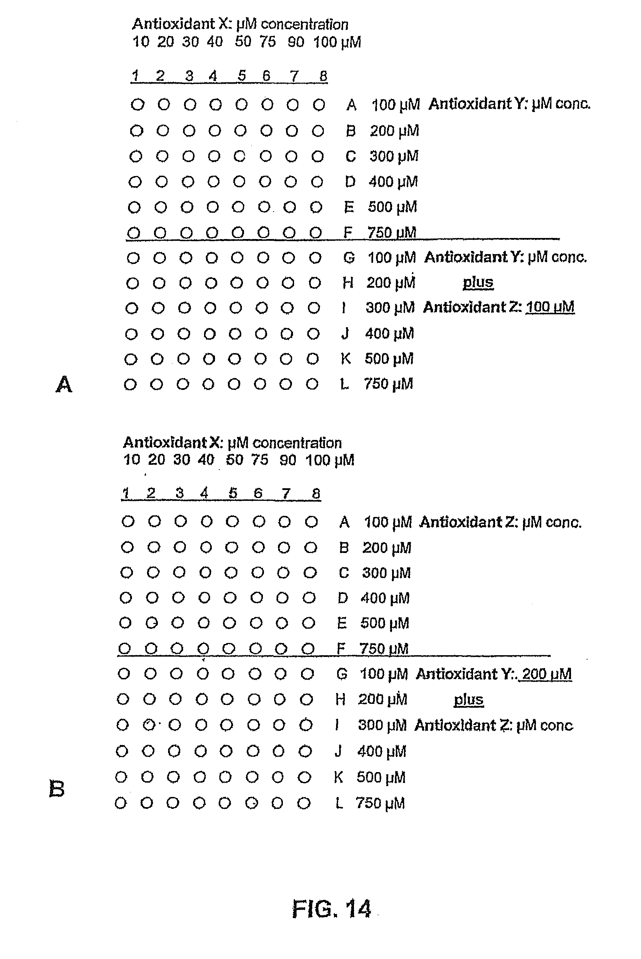

A format particularly well suited for high-throughput screening of test antioxidant agents is a multi-well plate. An exemplary system for performance of the method is a 96-well format, as shown schematically in Figs. 14A and 14B.

As discussed, the biomarkers of OS in the cells exhibit a quantitative response to oxidative stress, such as a reproducible, quantitative increase in the level of gene expression (up-regulation) in response to incremental changes in the concentration of the inducer of oxidative stress (i.e., a dose-response relationship between concentration of inducer and level of induced gene expression). Using the system, baseline levels of induction of gene expression in response to known levels of an OS inducer are established or known, and this value(s) serves as a control for comparison with levels of gene expression detected under conditions of induced OS in the presence of one or more test antioxidant agents.

A multi-well format is convenient for performing assays that include a plurality of replicate cultures to be compared. Referring to Figs. 14A and 14B, one variation of the method is performed in an "antioxidant screening matrix" suitable for simultaneous testing of a plurality of antioxidant agents (agents "X," "Y," and "Z" in the drawings) and/or combinations thereof, at several different concentrations. Many different experimental setups can be envisioned, as will be appreciated by those of skill in the art. As but one example, the arrangement shown in the top portion of Fig. 14A (columns 1-8; rows A-F) is a matrix designed to test effects of antioxidant agent "X" (in eight concentrations) in combination with antioxidant agent "Y" (in six concentrations).

The lower half of the matrix (columns 1-8; rows G-L) permits assessment of the effects of a combination of three antioxidants, i.e., agents "X," "Y," and "Z." In the example shown in Fig. 14A (lower), the same eight concentrations of agent "X" and 6 concentrations of agent "Y" are used, with a single concentration (in this case 100 µM) of agent "Z" further added to the wells in rows G-L.

Referring now to Fig. 14B, in the example matrix shown in the upper half of the Figure, the same eight concentrations of agent "X" as in Fig. 14A are used. This agent is combined for testing with six concentrations of agent "Z." In the lower half of the matrix, the same combinations of agents "X" and "Z" are tested as in the upper half, with the further addition of agent "Y" at a single concentration (in this case, 200 µM).

For determining whether or not an agent is a prophylactic antioxidant agent, or for determining the optimal combination of antioxidant agents for formulating an antioxidant nutritional/pharmaceutical product, the level of expression of at least one marker of oxidative stress in the cells is determined in the various groups. A quantitative difference in expression level of the marker between cells in the first (OS-only) and second (OS + antioxidant agent) test groups (typically, a lowering of expression of a gene up-regulated by oxidative stress) indicates that the agent is a prophylactic antioxidant agent. The most efficacious combinations of antioxidant agents, and the concentrations of each, can be determined on the basis of optimized change (typically reduction) of expression of the marker gene(s) of interest.

Methods for quantifying changes in gene expression are well known in the art of molecular biology. See, generally Sambrook, and Ausubel, *supra.* A preferred quantitative method for analysis of gene expression levels is quantitative real-time PCR (qPCR). Following extraction of RNA from the cultures on the tissue culture dishes, levels of gene transcription of the marker genes are detected by reverse transcription and amplification of marker gene sequences with primers specific for genes that respond to oxidative stress, preferably in a quantitative manner. As shown below, exemplary OS marker genes for analysis in retinal cells (RPE cells) include, but are not limited to *FosB, JunB, EGR-1* and heme oxygenase *(HO-1).* Further details pertaining to amplification of these transcripts and analysis by qPCR are provided in the Examples section, *infra.*

### Antioxidant Nutritional or Pharmaceutical Compositions

The systems and methods of the invention are believed to be especially useful for identifying new antioxidant agents (in particular those having beneficial effects on particular cell types in need of antioxidant therapy), and for developing new, more effective nutritional or pharmaceutical formulations of known antioxidants comprising new combinations of agents, and/or optimized concentrations thereof.

Accordingly, yet a further aspect of the invention is a nutritional or pharmaceutical composition comprising an antioxidant agent or agents, identified according to the above methods using a cell-based assay of oxidative stress.

Agents that reduce oxidative stress, identified by the methods listed *supra,* may be formulated into nutritional or pharmaceutical preparations for administration to mammals for prevention or treatment of disorders in which oxidative species have been implicated. In a preferred embodiment, the mammal is a human.

Compositions comprising a compound of the invention formulated in a compatible pharmaceutical carrier may be prepared, packaged, and labeled for treatment. As used herein, the term "pharmaceutical carrier" or "pharmaceutically acceptable carrier" refers to a carrier medium that does not interfere with the effectiveness of the biological activity of the active ingredient, is chemically inert, and is not toxic to the patient or animal subject to whom it is administered. A carrier of choice also may protect the prophylactic antioxidant(s) during preparation, shelf life, administration, or targeting from degradation by environmental exposure to oxygen, reactive oxygen species, intracellular oxidative stress or other unwanted side reactions such as hydrolysis, degradation, or functionalization. The carrier may assist in targeting to tissue, cell, organelle, or biomolecule.

If the complex is water-soluble, then it may be formulated in an appropriate buffer, for example, phosphate buffered saline or other physiologically compatible solutions. Alternatively, if the resulting complex has poor solubility in aqueous solvents, then it may be formulated with a surfactant such as Tween, or a polymer such as polyethylene glycol or a biologically innocuous solvent such as a simple alcohol or ester.

The compounds and their physiologically acceptable solvates may be formulated for administration by inhalation or insufflation (either through the mouth or the nose) or topical, oral, buccal, parenteral, rectal administration or, in the case of tumors, directly injected into a solid tumor.

For oral administration, the pharmaceutical preparation may be in liquid form, for example, solutions, syrups or suspensions, or may be presented as a drug or nutritional product for reconstitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g. almond oil, oily esters, or fractionated vegetable oils); and preservatives (e.g. methyl or propyl-p-hydroxybenzoates or sorbic acid).

The nutritional or pharmaceutical compositions may take the form of, for example, tablets or capsules or softgels prepared by conventional means with pharmaceutically acceptable excipients such as binding agents (e.g., pregelatinized maize starch, polyvinyl pyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Preparations for oral administration may be suitably formulated to give controlled release of the active compound. Capsules or encapsulations may be improved by substituting gelatin-free polymer materials, such as alginate, in order to eliminate risk from encephalopathies.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient(s) may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides. The compounds may also be formulated as a topical application, such as a cream or lotion.

As appropriate compositions for topical application there may be cited all compositions usually, employed for topically administering therapeutics, for example creams, jellies, dressings, shampoos, tinctures, pastes, ointments, salves, powders, liquid or semiliquid formulations and the like. Application of the compositions may be by aerosol, for example with a propellent such as nitrogen, carbon dioxide, a freon, or without a propellent such as a pump spray, drops, lotions, or a semisolid such as a thickened composition which can be applied by a swab. In particular compositions, semisolid compositions such as salves, creams, pastes, jellies, emollients, ointments and the like will conveniently be used. Compositions formulated for topical application to the skin can be incorporated into a patch, such as a transdermal patch. The compositions can also be formulated in a collagen matrix such as artificial skin. As used herein, the expressions "application to the skin," "topical application," and "application to a body surface" are intended to encompass application of the composition to either an intact body surface or to wounded body surface. For example, topical application to intact skin (for example, for a cosmetic purpose) would involve application to the epidermis, whereas topical application to a third degree burn would involve application to deeper subepidermal and even dermal tissues exposed to the surface after the burn.

Some preferred embodiments of the antioxidant agents are formulated for application to the eye. As used herein, the term "application to the eye" encompasses topical application, for example of an eyedrop or emollient formulation to the cornea or sclera of the eye, and further includes application of an appropriately formulated pharmaceutical preparation to interior structures of the eye, such as the retina or RPE, for example during ophthalmic surgery.

In addition to the formulations described previously, the compounds may also be formulated as a depot preparation. Such long-acting formulations may be administered by implantation (for example, subcutaneously or intramuscularly) or by intramuscular injection. Thus, for example, the compounds may be formulated with suitable polymeric or hydrophobic materials (for example, as an emulsion in an acceptable oil) or ion exchange resins, or as sparingly soluble derivatives, for example, as a sparingly soluble salt. Liposomes and emulsions are well known examples of delivery vehicles or carriers for hydrophilic drugs.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

The invention also provides kits for carrying out the therapeutic regimens of the invention. Such kits comprise in one or more containers therapeutically or prophylactically effective amounts of the compositions in pharmaceutically acceptable form. The composition in a vial of a kit of the invention may be in the form of a pharmaceutically acceptable solution, e.g., in combination with sterile saline, dextrose solution, or buffered solution, or other pharmaceutically acceptable sterile fluid. Alternatively, the complex may be lyophilized or desiccated; in this instance, the kit optionally further comprises in a container a pharmaceutically acceptable solution (e.g., saline, dextrose solution, etc.), preferably sterile, to reconstitute the complex to form a solution for injection purposes.

In another embodiment, a kit of the invention further comprises a needle or syringe, preferably packaged in sterile form, for injecting the complex, and/or a packaged alcohol pad. Instructions are optionally included for administration of compositions by a clinician or by the patient.

The sites and mechanisms of administration are of considerable variability. For small transportable prophylactic antioxidants, topical administration may be appropriate. For higher-molecular-weight prophylactic antioxidants the location of either an injected bolus or an implant can be variable requiring optimization and coordination of concentration, duration and location. The latter could range from topical, for example with transport enhancement, to intraocular, or an intermediate location such as *sub-tenons* or juxtascleral. And in some circumstances localized and targeted delivery can be achieved by oral administration. New technologies permitting delivery from intraocular devices or specialized reservoir-based contact lenses may be suitable. Ultimately, efficiency and therapeutic index probably will dictate the selection of route and mechanism of administration.

### EXAMPLES

The invention is further illustrated by reference to the following non-limiting Examples.

### Materials and Methods:

The following materials and methods generally can be used in the practice of the invention, and are further described in particular Examples below.

### Cell Culture Conditions:

To evaluate the net influence of rinse conditions (also termed "wash" conditions) on OS-induced changes in RPE cell gene expression, ARPE19 (human RPE cell line) cells were grown in 6-well plates in DMEM/F12 (with 10% FBS) until confluent. The DMEM/F12 media was removed and the cells were re-fed with 3 ml defined NR-1 media (37°C) (BioSource, Camarillo, CA) for 3 days to equilibrate gene expression. Parallel cultures were used as a source of "conditioned" NR-1 media ("CM")for the rinses described below. Conditioned, defined media was used to minimize induction of gene expression by higher concentrations of growth factors, glucose, and other potentially stimulating components in "fresh" media and FBS. The effect of rinse conditions alone on RPE gene expression (independent of OS) was examined by isolating RNA at specified time points (1-hour and 4-hours) after rinsing the cells at an arbitrary "0 time point" using the protocols listed in Table 1. Table 1. Rinse conditions for assay of oxidative stress No touch Direct RNA isolation from cells without rinsing. Media replacement Cells rinsed once with conditioned NR-1 media (37°C) after 1 hour of OS. RNA isolated at subsequent time points without additional rinsing PBS Rinse Cells rinsed twice with PBS after 1 hour of OS then re-cultured in conditioned NR-1 media (37°C). RNA isolated as above. Media Rinse 1 Cells rinsed twice with conditioned NR-1 media (37°C) after 1 hour of OS, then re-cultured in conditioned NR-1 media (37°C). RNA isolated as above. Media Rinse 2 Cells rinsed twice with conditioned NR-1 media (37°C) as above, by aspirating only 2/3 volume of media after 1 hour of OS, then re-cultured in conditioned NR-1 media

**Table1. Rinse conditions for assay of oxidative stress**

| | |
|---|---|
| No touch | Direct Rna isolation from cells without rinsing. |
| Media replacement | Cells rinsed once with conditioned NR-1 media (37°C) after 1 hour of OS. RNA isolated at subsequent time points without additional rinsing. |
| PBS Rinse | Cells rinsed twice with PBS after 1 hour of OS then re-cultured in conditioned NR-1 media (37°C). RNA isolated as above. |
| Media Rinse1 | Cells rinsed twice with conditioned NR-1 media (37°C) after 1 hour of OS, then re-cultured in conditioned NR-1 media (37°C). RNA isolated as above. |
| Media Rinse2 | Cells rinsed twice with conditioned NR-1 media (37°C) as above, by aspirating only 2/3 volume of media after 1 hour of OS, then re-cultured in conditioned NR-1 media |

### Induction of Oxidative Stress (OS):

For OS studies, ARPE19 cells were incubated for 1 hour at 37°C in conditioned NR-1 media containing 50-500 µM H₂0₂, Cells were either not rinsed (1-, 2-, and 4-hour isolations) or rinsed using the Media Rinse 2 method (4-, 8-, and 24-hour isolations). At designated time-points, NR-1 medium was aspirated and the total cellular RNA was immediately isolated in TRI reagent (Sigma). Single wells were used in each plate to minimize any influence of temperature shifts on gene expression. All procedures were performed in a dim red light environment to reduce any influence of light on gene expression. RNA was isolated either before the onset of OS (0-hour control), or at 1, 2, 4, 8, or 24 hours after the start of the 1-hour OS treatment.

To determine the influence of shearing forces on the RPE cells, aspiration of media was performed either manually or by vacuum extraction. All additions of culture media and solutions were performed using a manual pipettor.

### RNA Isolation and Quantitative PCR:

### Primer Design:

Generally, gene-specific primers can be designed from the library of Human Genome U133 Plus 2.0 Array 3'-probe sets (Affymetrix) using Primer Express software v1.5a (ABI). Genes of interest in studies of oxidative stress include but are not limited to: catalase (CAT), superoxide dismutase (SOD 1-3), glutathione synthetase (GSS), FBJ murine osteosarcoma viral oncogene homolog B (FosB), jun B proto-oncogene (JunB), heme oxygenase-1 (HO-1) and early growth response factor-1 (EGR-1). For a further listing of molecular markers of oxidative stress, see also Example 6, and Table 2, *infra.*

PCR primers useful to amplify these genes are listed below:
Fos B
   forward: 5'- GTG TGA GCG CTT CTG CAG C - 3' (SEQ ID NO:1)
   reverse: 5'- CCA ATT CAA CGG CTC GCT T - 3' (SEQ ID NO:2)
JunB
   forward: 5'- CCT TCC ACC TCG ACG TTT ACA - 3' (SEQ ID NO:3)
   reverse: 5'- AAT CGA GTC TGT TTC CAG CAG AA - 3' (SEQ ID NO:4)
EGR-1
   forward: 5'- TTT CAC GTC TTG GTG CCT TTT - 3' (SEQ ID NO:5)
   reverse: 5'- CCC TCA CAA TTG CAC ATG TCA - 3' (SEQ ID NO:6)
HO-1
   forward: 5'- GCC TAT GGC ATC TTC CCC A - 3' (SEQ ID NO:7)
   reverse: 5'- TTC GCC CCC TCT GAA GTT TA - 3' (SEQ ID NO:8)
SOD2
   forward: 5' - TGC TGC TTG TCC AAA TCA GG - 3' (SEQ ID NO:9)
   reverse: 5'- CAC ACA TCA ATC CCC AGC AGT - 3' (SEQ ID NO:10)
GSS
   forward: 5'- AAG GTC CAT GAA CCC TGC C - 3' (SEQ ID NO:11)
   reverse: 5'- GGC ACT GGA ACC TGC TGA AA - 3' (SEQ ID NO:12)
CAT
   forward: 5'- CAG GTG CGG GCA TTC TAT GT - 3' (SEQ ID NO:13)
   reverse: 5'- CCG GCA ATG TTC TCA CAC AG - 3' (SEQ ID NO:14)

### RNA Isolation and Real-Time Polymerase Chain Reaction (PCR) Analysis:

Total cellular RNA is isolated by direct lysis in TRI reagent following aspiration of the media. RNA samples are cleaned of genomic DNA contaminants by treatment with DNAse I (Ambion). The RNA is stored in DNAse-free water at 0.2µg/µl. About one µg of RNA is reversed transcribed (RT) in 20µl of reaction volume using an RT System (Promega). The RT product is diluted 1:5 and real-time PCR is performed, for example using the ABI PRISM 7700.

### Example 1- Optimizing Culture Conditions for Demonstrating Quantitative OS-Induced Gene Expression

In preliminary studies, fluctuations in gene expression were observed in response to the tissue culture protocols used for the cell-based assays. This example describes a systematic analysis to identify the factors responsible for non-specific cellular responses. Major inducing factors are identified and techniques to minimize them in quantitative studies are described.

### Methods:

Human ARPE-19 cells were seeded into 6-well tissue culture plates and grown to confluence. After reaching confluence, the cells were cultured for three days in defined NR-1 medium to stabilize gene expression. RNA was isolated from the cells at 0, 1, 4, and 24 hours following different rinse conditions. These included: i) no touch control, ii) manual pipette aspiration followed by media replacement with conditioned media, iii) vacuum aspiration followed by PBS rinse, then media replacement with conditioned media, iv) vacuum aspiration followed by conditioned media rinse, then media replacement with conditioned media, and v) manual pipette aspiration of half of the media (x2), followed by media replacement with conditioned media. Total cellular RNA was isolated at each time point noted. Gene expression was quantified using real-time RT-PCR methods.

### Results:

The results are summarized as follows:
Standard vacuum aspiration followed by rinsing with either PBS or conditioned media induced very large quantitative changes in the expression *of FosB, JunB,* and *EGR-1* (the most responsive genes) within one hour. The effect of rinse conditions alone on the expression of transcription factors *FosB, JunB,* and *EGR-1.* Rinsing the cells with PBS or conditioned media induced very large increases in gene expression within one hour. The peak level of induction increased by as much as 64- to 128-fold within 1 hour, depending on the rinse conditions. This effect was significantly reduced by more gentle rinsing methods but expression still increased by as much as 8-fold after 4 hours. All genes returned to basal levels of expression by 24 hours.

The effect of media exchange was much less for other genes; for example only a small effect on HO-1 was observed after 4 hours. Only with elimination of all manipulations, demonstrated using a no-touch control, were all changes in gene expression eliminated (except in the case of *EGR-1*). Overall, these studies demonstrated that routine tissue culture methods can induce significant changes in gene expression in human RPE cell cultures. Without intending to be bound by theory, it is believed that this may be due to mechanical sheer stress, transient cell drying, changes in osmolarity, or other causes. The potential confounding effects of such culture methods used in studies of OS must be carefully controlled in order to accurately determine OS-specific responses.

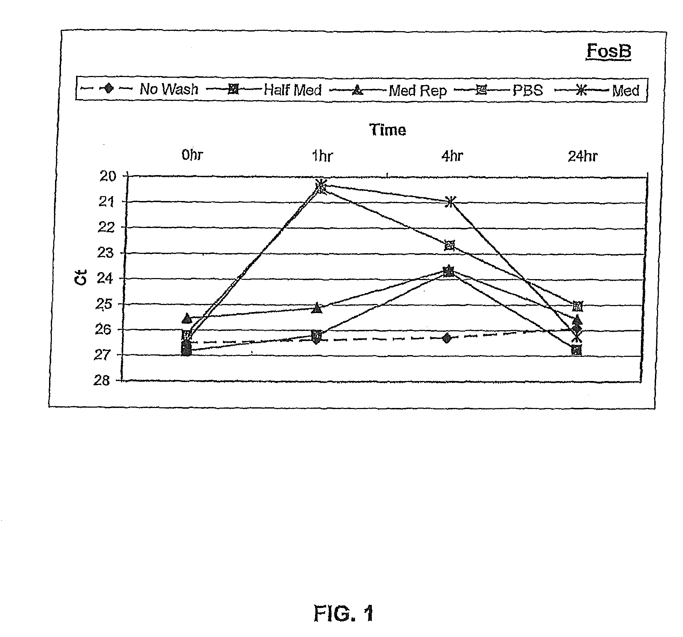

Results of studies with the individual genes are described in further detail below. Referring to Figure 1, the effect of tissue culture rinse conditions on *FosB* gene expression is shown. Significantly, Figure 1 demonstrates that rinsing the cells with buffered saline or conditioned media alone induces *FosB* expression. Progressively milder rinse conditions reduce this effect at 1 and 4 hours, but do not eliminate it. In the Figure, the rinse conditions are as follows: 1) No rinse; "no wash" (immediate RNA isolation without manipulation); 2) Half media; "Half Med" (limited removal of media and replacement with conditioned media from the same well); 3) Media replacement; "Med Rep" (gentle removal with a pipettor and dropwise addition of conditioned media); 4) phosphate buffered saline; "PBS" (aspiration followed by rinse with phosphate buffered saline, then addition of conditioned media); and 5) Media replacement + CM; "Med" (aspiration followed by rinsing with conditioned media, then addition of conditioned media).

The data depicted in Figure 1 are presented on an inverted scale, i.e., each decrease in the control (Ct) value represents a 2-fold increase in transcription of the *FosB* mRNA. For example, as can be seen, transcription increases 64-fold at 1 hour in cells rinsed with buffered saline (PBS curve, ΔCt = 6, or 2⁶). The data clearly show that the "no rinse" technique has no effect on gene expression at any time point (dotted line). The gentler "half media" and "media replacement" techniques have no significant effect on gene expression at 1 hour, but do induce an increase in gene expression at the 4 hour time point, which returns to baseline after 24 hours. Conversely, the PBS and media rinse techniques strongly induce *FosB* gene expression at one hour. These effects were sustained for 4 hours and then returned to baseline after 24 hours. These data indicate that the *FosB* transcription factor is exquisitely sensitive to media conditions and techniques that are routinely used in tissue culture. To our knowledge, this important methodological finding has not previously been appreciated or reported.

### Example 2- Dose-dependent Responses to Oxidative Stress (OS)

This example shows that using optimized culture conditions as described in the above Example, accurate quantification of OS-specific changes in gene expression can be achieved using the cell-based assay.

### Methods:

Assays of oxidative stress as described above with RPE cells were performed using the "no touch" technique, to determine whether OS-specific responses of *FosB* gene expression occurred during the first 8 hours after induction of stress. In addition, a dose-response study of *FosB* gene expression was performed to determine whether the degree of OS could be quantified using this molecular response.

OS-induced transcriptional responses were measured by quantitative RT-PCR (qPCR) following RNA isolation using the "no touch" method at 1 hour and 4 hours after OS, and at 8 hours after OS (H₂O₂ removed by "Half med" rinse after 1 hour)

### Results:

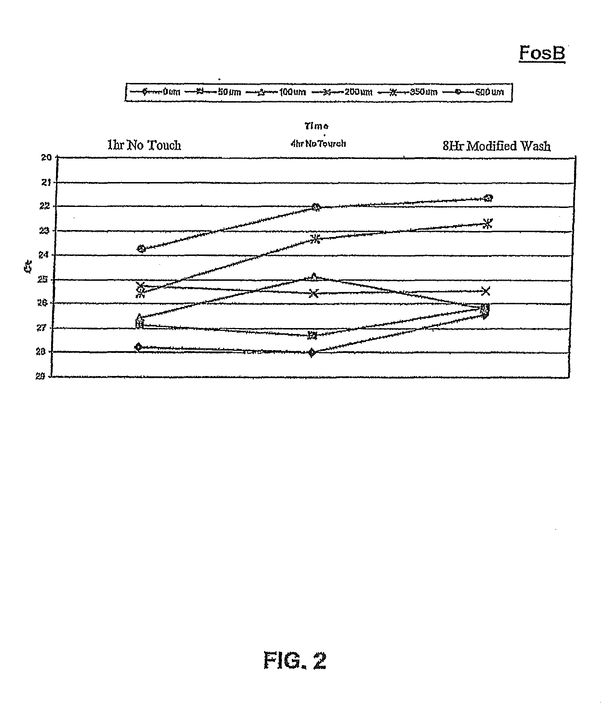

Referring to Figure 2, the data from qPCR showed a significant and stress-specific response of *FosB* transcription in the RPE following OS. As can be seen, the control (Ct) value for *FosB* expression at time 0 was 28. *FosB* transcription increased 16-fold at 1 hour in cells treated with 500 µM H₂O₂ (ΔCt = 4, or 2⁴). *FosB* transcription increased 64-fold after 4 hours for the same level of OS (ΔCt = 6, or 2⁶). The data clearly demonstrates that the OS-specific increase in *FosB* transcription is dose-dependent at both 1 and 4 hours after OS under the described experimental conditions, except for only a slight inversion of the 100 and 200 µM curves at 4-hours (Figure 2). This dose-dependent response is stable and preserved after 8 hours, although there is a small rinse-induced increase in expression at 8 hours. This rinse effect appears to account for the slight increase in transcription seen at 8 hours, relative to the 4-hour time point. We interpret this to show that *FosB* expression after 4 hours is effectively stable for an additional 4 hours.

### Example 3- Expression Profiles of Biomarkers in Cell-based Assay of Oxidative Stress

This Example describes effects of culture conditions on expression a several biomarkers, i.e., *JunB, EGR-1* and *HO-1* in a cell-based assay in accordance with the invention.

### Methods:

Quantitative *JunB* gene expression studies were performed by qPCR using the cell-based assay with RPE cells and the "no-touch" and modified rinse conditions as described above.

### Results:

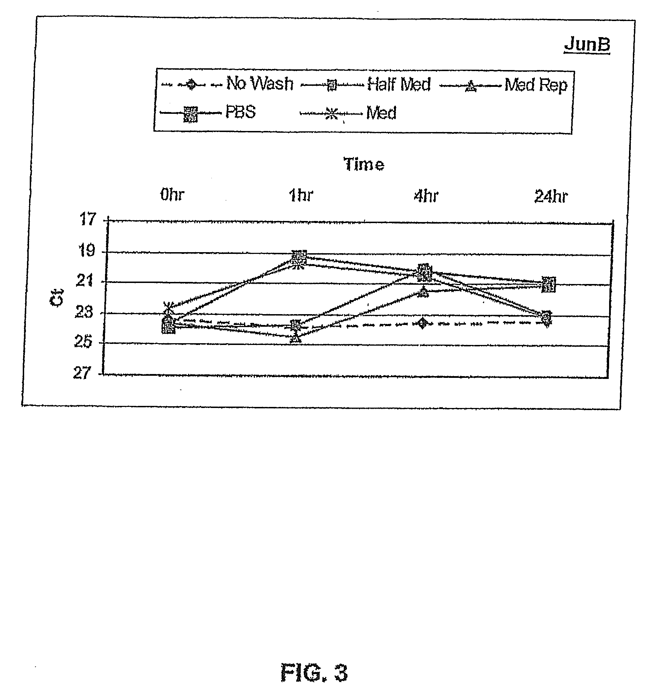

Referring to Figure 3, a method-induced increase in *JunB* transcription was observed at both 1 and 4 hours after subjecting the cultures to OS. More specifically, FIG. 3 shows the effect of tissue culture rinse conditions on *JunB* gene expression in RPE cells. Rinsing the cells with buffered saline or conditioned media alone, in absence of an exogenous stress-inducing agent, induced *JunB* expression in these cells. As with *FosB,* a similar response pattern was seen for *JunB* in which gene expression increased by 8- to 16-fold 1 hour after a standard rinse. Expression also increased to similar levels 4 hours after gentle rinsing (Figure 3; P<0.05 to <0.001). There was no change in gene expression in the "no touch" group. *JunB* expression remained elevated (8-fold) for up to 24 hours after the less gentle rinse conditions.

These studies demonstrate that the significant increase in *JunB* gene expression seen in both qPCR and in microarray studies can be due to tissue culture manipulation rather than OS. However, as in the case of *FosB,* this undesired effect could be eliminated using a "no-touch" approach as described above that did not include a change in the media before RNA extraction. Similar studies were conducted to study effects of culture conditions on EGR-1 and HO-1 gene expression. The results showed a strong response pattern for *EGR-1* in which gene expression increased by 64- to 128-fold 1 hour after a standard rinse. Results of quantitative studies showed that expression levels remained elevated at 4 hours but returned toward baseline levels after 24 hours (P<0.05 to <0.001). *EGR-1* expression was seen to increase up to 4-fold under control conditions in which *FosB* and *JunB* expression levels were stable. The reason for this transient increase in expression under tightly controlled conditions was not determined.

Unlike the above transcription factors, there was no demonstrable effect of rinse conditions on *HO-1* expression after 1 hour. However, a 2-4-fold increase in expression was seen 4 hours after both standard and gentle rinses *(P<0.05). HO-1* expression returned to baseline levels after 24 hours.

### Example 4- Comparative Analysis of Quantitative Gene Regulation by Oxidative Stress (OS)

This example describes studies demonstrating comparative effects of oxidative stress in RPE cells with respect to expression of genes in several families of transcription factors and/or protective chaperone proteins (i.e., *Fos, Jun, ATF, HO-1,* and crystallin).

### Materials and Methods:

### 1. Tissue Culture Conditions

Human retinal pigment epithelial (RPE) cells (ARPE-19) were cultured in DMEM/F12 media supplemented with 10% fetal bovine serum (FBS, Atlanta Biologicals, Norcross, GA) plus L-glutamine, penicillin, and streptomycin in an atmosphere of humidified 95% air and 5% CO2 at 37°C until confluent in single wells of 6-well plates. The cells were initially grown in DMEM/F12 with FBS to reduce the time to reach confluence. Cells were then rinsed and fed with NR-1 media (BioSource, Camarillo, CA). NR-1 is a chemically defined tissue culture medium supplemented with EGF, insulin, hydrocortisone, and transferrin. After feeding with NR-1, the confluent cells were cultured for 3 days to stabilize gene expression.

As shown above, rinsing cells *in vitro* with buffered saline or media can induce profound increases in the expression of IEG transcription factors. To minimize the potential effects of temperature and CO₂ fluctuations on gene expression when cells are removed from the incubator, only a single well was used in each tissue culture plate. All procedures were performed under dim red light illumination to minimize the potential influence of light on RPE gene expression.

Oxidative stress was induced by the addition of stock H₂O₂ solution to the NR-1 media to bring the final media concentration to the desired level (0 to 500 µM H₂O₂). The OS was not rinsed from the culture; rather, the NR-1 medium was aspirated and TRI Reagent (Sigma, St. Louis, MO) was immediately added to lyse the cells and stabilize the RNA at the designated time-points. An OS of 500 µM H₂O₂ for up to 4-hours is 55% of the dose required to induce 10% cell death (LD₁₀) in our confluent cell culture assays (MTT data not shown).

### 2. RNA Isolation

Total cellular RNA was isolated from the cells at time points 0, 1, and 4 hours using TRI reagent as described above using the protocol recommended by the manufacturer. The RNA was cleaned of trace DNA contaminants by treatment with DNA-Free (Ambion, Austin, TX) and the concentration was measured by spectrophotometry. The purified RNA was dissolved in DEPC-treated double distilled water at the concentration of 0.2 mg/ml. and stored at -80°C.

### 3. PCR Primers

Genes chosen for real-time PCR analysis from pilot microarray data were selected from the Human Genome U133 Plus 2.0 Array library (Affymetrix, Santa Clara, CA). These included:
FBJ murine osteosarcoma viral oncogene homolog B (FosB):
   forward: 5'- GTG TGA GCG CTT CTG CAG C - 3' (SEQ ID NO:1);
   reverse: 5'- CCA ATT CAA CGG CTC GCT T- 3' (SEQ ID NO:2);
jun B proto-oncogene *(JunB):*
   forward: 5'- CCT TCC ACC TCG ACG TTT ACA - 3' (SEQ ID NO:3);
   reverse: 5'- AAT CGA GTC TGT TTC CAG CAG AA - 3' (SEQ ID NO:4);
superoxide dismutase-2 *(SOD2):*
   forward, 5'- TGC TGC TTG TCC AAA TCA GG - 3' (SEQ ID NO:9);
   reverse, 5'- CAC ACA TCA ATC CCC AGC AGT - 3' (SEQ ID NO:10).
glutathione synthetase *(GSS):*
   forward, 5'- AAG GTC CAT GAA CCC TGC C - 3' (SEQ ID NO:11);
   reverse, 5'- GGC ACT GGA ACC TGC TGA AA - 3' (SEQ ID NO:12);
catalase (CAT):
   forward, 5'- CAG GTG CGG GCA TTC TAT GT - 3' (SEQ ID NO:13);
   reverse, 5'- CCG GCA ATG TTC TCA CAC AG - 3' (SEQ ID NO:14);
heme oxygenase-1 *(HO-1):*
   forward, 5' - TTT CAC GTC TTG GTG CCT TTT - 3' (SEQ ID NO:15);
   reverse, 5'- CCC TCA CAA TTG CAC ATG TCA - 3' (SEQ ID NO:16)
insulin-like growth factor 1 (*IGF 1*):
   forward, 5' - CCA CCC TTG AGA ATC CTT CCT - 3' (SEQ ID NO:17);
   reverse, 5' - GGA GCC ACA GAG CAT GAG ATG - 3' (SEQ ID NO:18);
β-action:
   forward, 5' - CAC CCT GAA GTA CCC CAT CG - 3' (SEQ ID NO:19);
   reverse, 5' - TGC CAG ATT TTC TCC ATG TCG - 3' (SEQ ID NO:20).

Target sequences for these genes were obtained from GeneChip array information at NetAffx Analysis Center (http:/lwww.affymetrix.com/analysis/index.affx).

The primers (except those obtained from SuperArray) were designed using Primer Express® software v1.5a (ABI, Foster City, CA) and synthesized at IDT (Coralville, IA). Optimized primers for genes *ATF2, ATF3, CRYBA, CRYBB, CRYGS, cFos, FosL, GSR, MSRA, MGST1, TXN, TXNIP, NFE2L2,* and *HSP 27* (acronyms defmed below) were obtained commercially from SuperArray (Frederick, MD).

### 4. Real-time PCR

One µg of total cellular RNA was reversed transcribed (RT) in 20 µl of reaction volume using the Reverse Transcription System using the manufacturer's recommended protocol (Promega, Madison, WI). The RT product was diluted 1:5 with DNase-free water and quantitative PCR (qPCR) amplification was performed in 50µl of buffer containing 1x SYBR® Green PCR Master mix (ABI), optimized forward and reverse qPCR primers and 5µl of the 1:5 diluted RT product. The qPCR reaction was started at a 50°C hold for 2 minutes, then 95°C hold for 10 minutes, followed by 40 cycles of 95°C for 15 seconds, and 60°C for 1 minute. The reaction was performed using the ABI PRISM® 7700 Sequence Detection System.

The data were analyzed using a student T test with SPSS software (SPSS Inc., Chicago, IL). All values graphed are the mean and standard deviations of triplicate samples. Our previous studies demonstrated high quantitative fidelity for the expression levels in replicate tissue culture studies from experiment to experiment (Yang et al. JCB, in press). The standard deviation (S.D.) within a given experiment (e.g., JunB) ranged from about 0.05 to 0.37 cycles (n=6). CT values varied by less than half a cycle from experiment to experiment in stable control wells, but varied by up to one cycle when measured after stimulation. Due to the large quantitative fold-changes compared to the controls seen within experiments, replicate studies were not performed in every case. Selected data sets were replicated by repeat culture and RNA analysis where indicated, to clarify the variances in the qPCR data. Fold-changes are reported in integer form, closest to the nearest cycle for clarity.

### Results:

### 1. Fos Gene Expression

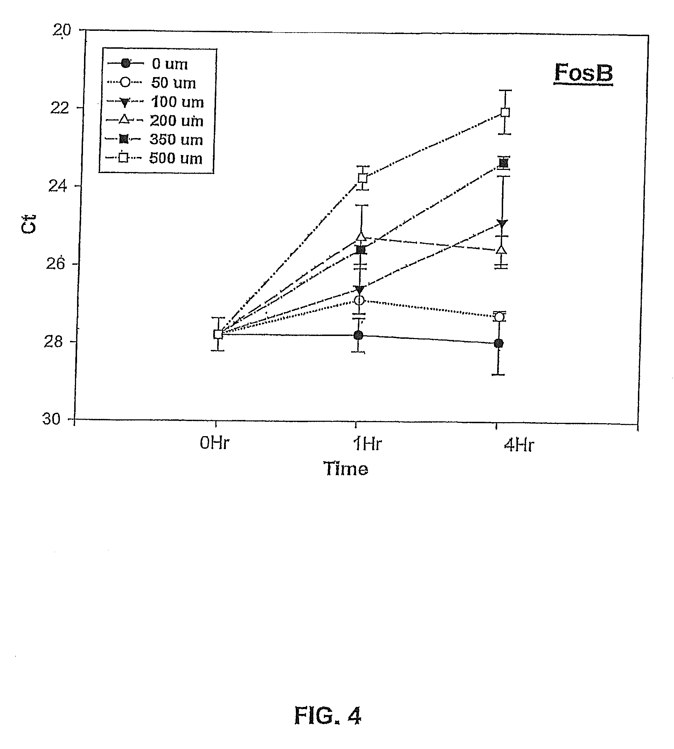

*FosB.* The expression of *FosB* was strongly induced in response to oxidative stress (OS) for up to 4 hours after the stress. Transcription increased 16-fold within 1 hour of OS and remained elevated, increasing to 64-fold by 4 hours, at which point it was maximal (P<0.001) (FIG.. 4). *FosB* expression returned to baseline levels within 24 hours after OS when the OS was removed from the media using our published methods. There was a strong, dose-dependent correlation between the levels of OS and the induced fold-changes in *FosB* transcription at both time points (FIG. 4).

*c-Fos* and *FosL*. Based on the results seen for *FosB,* we examined the OS responses of two other AP-1 family Fos genes, i.e., cellular Fos (*c-Fos*) and the Fos-like protein (*FosL*). Transcriptional responses to OS were quantified using the same method at 1 and 4 hours after OS.

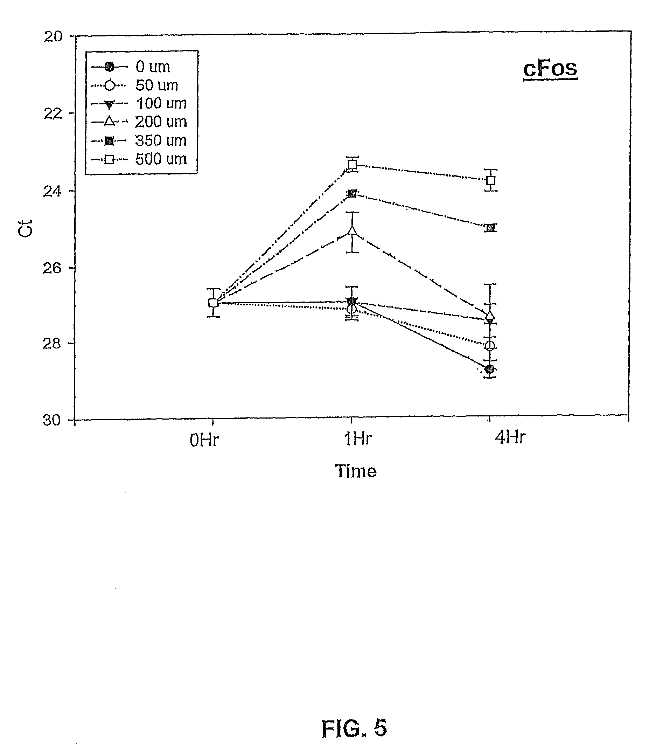

Referring now to FIG. 5, the *c-Fos* gene demonstrates an increase in transcription 1 hour after OS, similar to that seen for *FosB,* although quantitatively slightly less. There is an OS threshold of between about 100 and 200 µM H₂O₂ below which there is no detectable increase in *c-Fos* expression. Transcription of *c-Fos* increases up to 12-fold after 1 hour of OS and remains elevated with only a small decay after 4 hours to 8-fold elevation over controls (*P*<0.001). In addition to the threshold response seen, the transcriptional response above about 200 µM H₂O₂ is dose-dependent.

The stable temporal pattern of *c-Fos* activation differs from *FosB*, which continues to increase over the first 4 hours after stress. The transcription level of *c-Fos* peaks at 1 hour, but remains activated over 4 hours for the higher levels of OS. There is, however, a return to baseline levels of *c-Fos* expression over 4 hours in those cells exposed to the lowest level of OS in which a response was detected (200 µM H₂O₂).

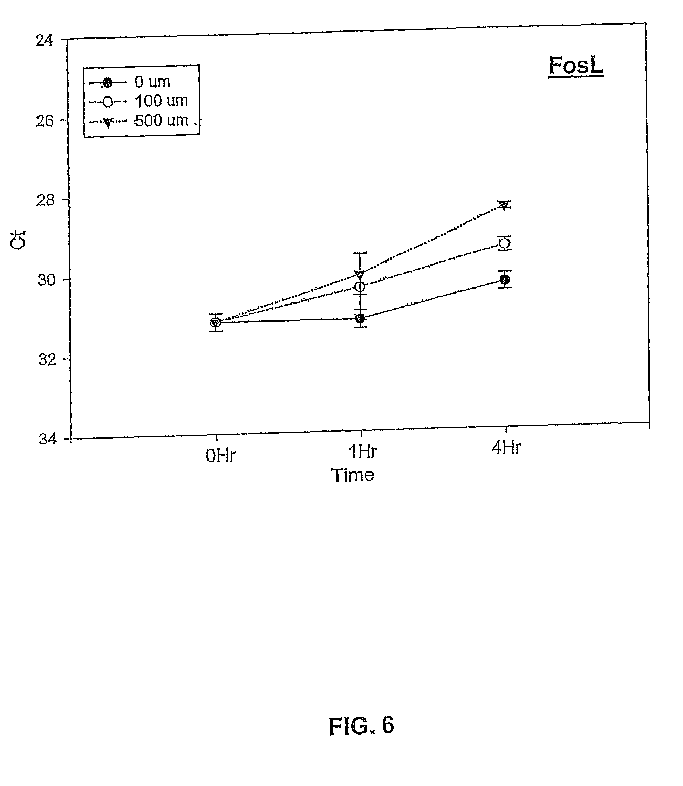

The *FosL* gene also shows a pattern of transcriptional activation after OS similar to *FosB,* but at significantly lowers levels (FIG 6). Transcription increases 2-fold after 1 hour of OS, at the two levels of OS tested (i.e., 100 µM and 500µMH₂O₂). As with *FosB, FosL* transcription continues to increase up to 4 hours after OS, at which time it is increased 3-fold compared to controls for the highest OS dose (*P*<0.005). The response is dose-dependent for the levels of OS tested; however, the increase in the level of transcription is significantly less than that seen for *FosB* and *c-Fos.*

### 2. Jun Gene Expression

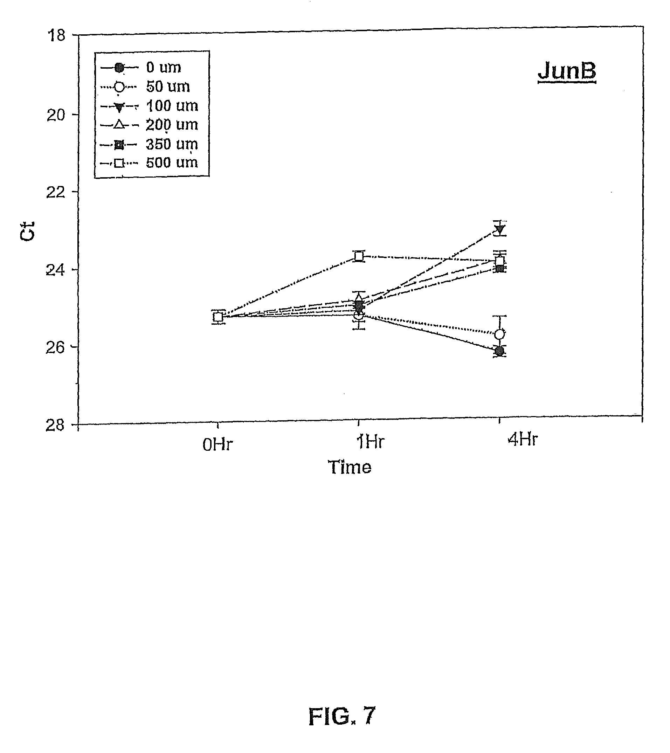

*JunB.* As noted above, transcription of *JunB* shows an increase of ~4-fold after 1 hour at the highest tested level of OS (i.e., 500µM H₂O₂), which remains stable and elevated over 4 hours (*P*<0.001), (FIG. 7). However, levels of OS below about 500 µM H₂O₂ do not induce *JunB* transcription at 1 hour, suggesting a high threshold response of *JunB* to OS. A different threshold response is seen at 4 hours for levels of OS ≥ about 100 µM H₂O₂. The level of transcriptional activation at 4 hours is ~4-fold and similar for all levels of OS ≥ about 100µM H₂O₂ (*P*<0.004). Although the responses seen at 1 hour (500 µMH₂O₂) and at 4 hours (≥100 µM) are dose-related, the response of *JunB* to OS appears to be a more qualitative threshold response.

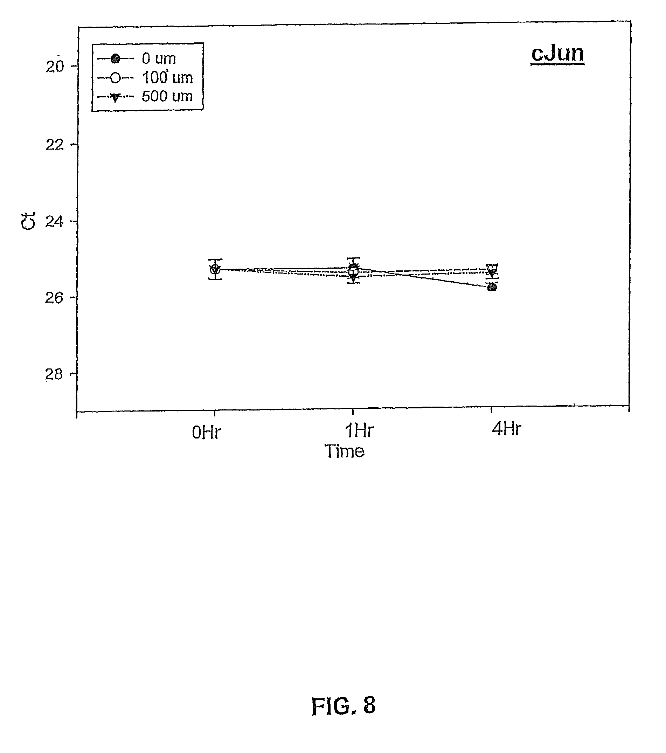

*c-Jun.* Unlike *JunB,* even high levels of OS do not increase transcription of the *c-Jun* gene under our experimental conditions (FIG. 8). No change is seen compared to controls at either the 1-hour or 4-hour time points even at the highest level of OS. Whereas *c-Fos* transcription appears to mirror that of *FosB* after OS, the same relationship does not hold true for *c-Jun* and *JunB.*

### 3. ATF Gene Expression

The quantitative nature of AP-1 transcription factor gene expression in OS responses in the RPE suggested to us that other genes associated with the AP-1 complex might also show quantitative gene regulation in OS. We therefore examined the expression of two AP-1 related genes, i.e., activating transcription factors *ATF2* and *ATF3.*

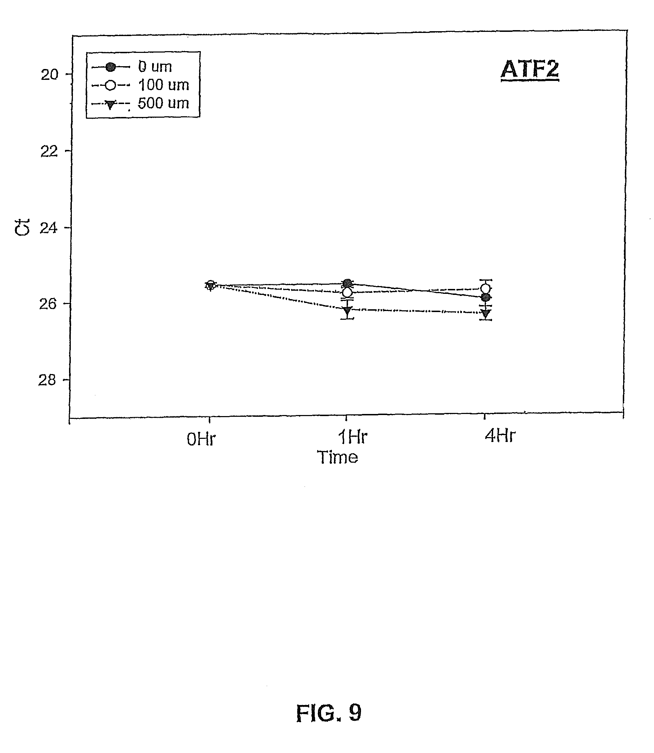

The results showed that high levels of oxidative stress do not increase transcription of the *ATF2* gene under our experimental conditions. Referring to FIG. 9, no change is seen compared with controls at either the 1-hour or 4-hour time points after OS.

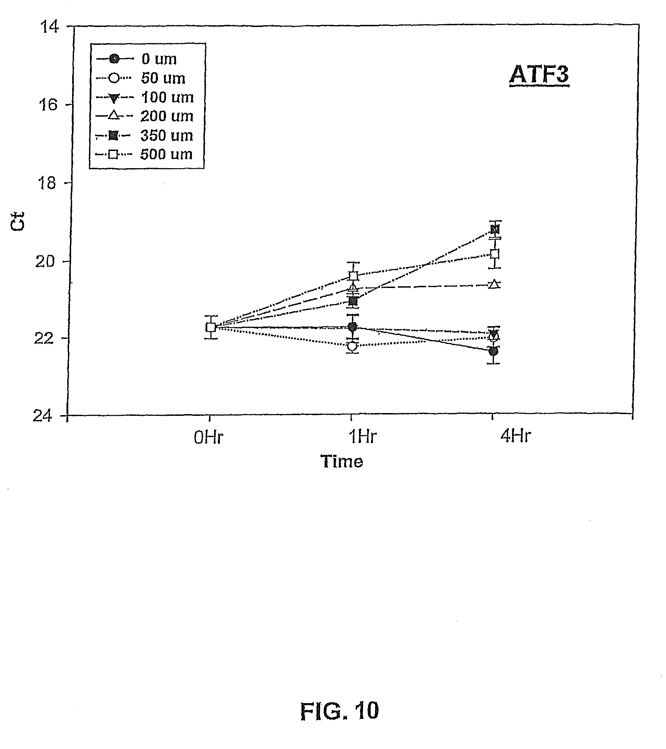

Conversely, *ATF3* gene transcription demonstrates a mixed threshold and somewhat dose-dependent response at both 1 hour and 4 hours after OS. Referring to FIG. 10, the increase in transcription is ~3-folk higher than levels of OS at 1 hour (*P*<0.022), and up to 8-fold higher for the highest level of OS (500 µM H₂O₂) at 4 hours (*P*<0.04). There is an OS threshold of between about 100 and 200 µM H₂O₂, below which there was no detectable increase in *ATF3* expression. The 100 µM H₂O₂ concentration threshold response is similar to the lower OS thresholds seen for both *c-Fos* and *JunB.* There is a dose-dependent response seen at 1 and 4 hours after the highest levels of OS (>200 M H₂O₂), although it is not strictly quantitative. Thus, *ATF3* demonstrates a mixed threshold and quantitative response to OS, similar to *c-Fos.*

To summarize, these results demonstrate that selected members of three AP-1 transcription factor complex gene families (i.e., *Fos, Jun, ATF*) are up-regulated in a dose-dependent fashion in response to increasing levels of OS. These include: *FosB, c-Fos,* (and to a lesser degree *FosL*); *JunB* (but not *c-Jun*); *and ATF3* (but not *ATF2*). Two of these genes, i.e., *c-Fos* and *ATF3* show a mixed threshold effect, where transcription is induced in a dose-dependent fashion, but only after exceeding a threshold level of OS. The *JunB* gene shows a threshold response that is quantitative at 1 and 4 hours after OS, but which does not appear to be dose-dependent.

### 4. Heme Oxygenase-1 (HO-1) Gene Expression

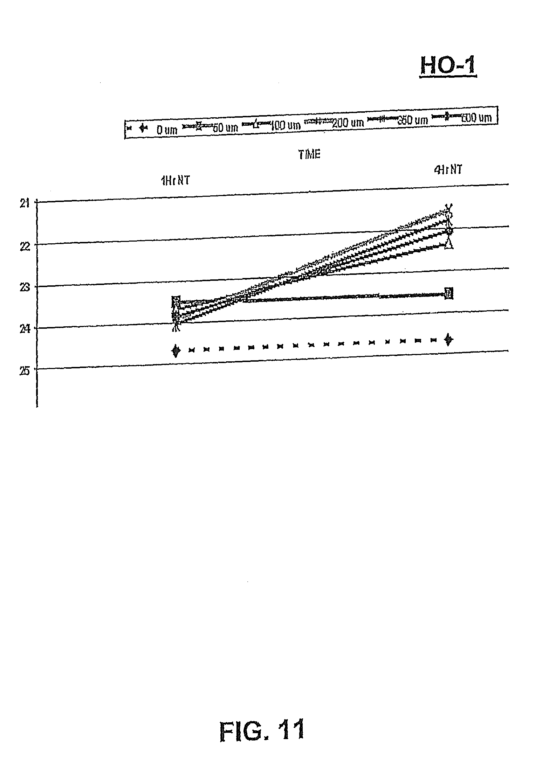

We quantified the expression of *HO-1* for up to 4 hours after exposure to OS. No significant response was seen after 1 hour. As shown in FIG. 11, there is a 3- to 6-fold increase in *HO-1* transcription seen for OS ≥100 µM. This appeared to be a purely qualitative threshold response and is not dose-dependent (*P*<0.018). This threshold response is similar to that seen for *JunB.*

### 5. Crystallin Gene Expression

The recognized role of the crystallin proteins as molecular chaperones that protect other proteins from stress-induced changes and degradation suggested to us that these genes would be potential candidates for OS-induced response genes in the RPE. We examined the transcription of 4 crystallin genes, i.e., *CRYAA, CRYBA, CRYBB,* and *CRYGS,* following OS.

We had preliminarily observed that *CRYAB* is not significantly induced by OS. Transcription of the above-identified crystallin genes was quantified at 1 and 4 hours in cells exposed to 100 µM and 500 µM H₂O₂ -induced OS, and compared to controls. The results confirmed that OS does not induce the *CRYAA, CRYBB,* and *CRYGS* crystallin genes in our assay.

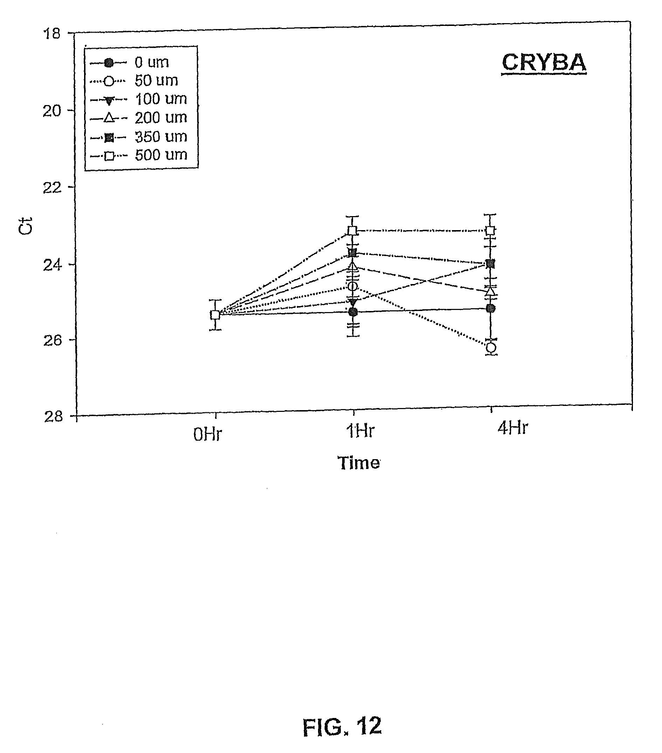

By contrast, the *CRYBA* gene demonstrates a dose-dependent and quantitative OS response at both the 1-hour and 4-hour time points (FIG. 12). Transcription of *CRYBA* after 500 µM H₂O₂ stress increases 4-fold over control levels and is maximal after 1 hour (*P*<0.01), remaining relatively stable over 4 hours. A quantitative relationship is seen at 1 and 4 hours after stress, for all levels of stress tested (FIG. 12).

### 6. Redox Gene Expression

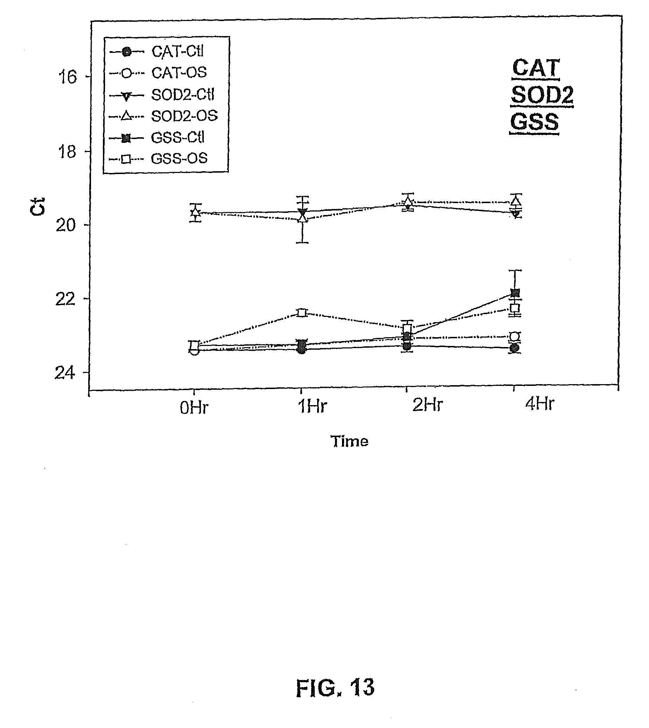

We quantified the expression of *GSS, CAT,* and *SOD-1* over 4-hours after OS to determine the temporal kinetics of the anticipated OS-response. With the exception of a small increase in *GSS* expression at one hour, there is no significant increase in the transcription in these redox genes during the first 4 hours after OS (FIG. 13).

We examined the expression of numerous other genes known to participate in regulating the redox state of the cell, to determine whether they were activated early following OS. These genes included: glutathione reductase (*GSR*), microsomal glutathione-S-transferase-1 (*MGST1*), methionine sulfoxide reductase A (*MSRA*), thioredoxin (*TXN*, and thioredoxin interacting protein (*TXNIP*). The only one of these genes to demonstrate a significant response to OS in the first 4 hours following stress is *TXN*, which shows a modest 2-fold response at 4 hours (*P*<0.014). Gene expression of β-actin is used as a control since this housekeeping gene is not anticipated to change to any significant degree in response to OS. As expected, no change in β-action expression is seen.

The foregoing results may be summarized as follows:

We have identified eight genes that are either moderately or strongly up-regulated in response to OS in RPE cells *in vitro.* These include: five genes associated with the AP-1 transcription factor family; two OS response genes; and a crystallin gene. The increase in transcription induced by OS is quantitative, showing dose-dependent, threshold or mixed responses, depending on the gene.

In contrast, known OS-response genes including *GSS, CAT, SOD-1, GSR, MGST1, MSRA,* and *TXNIP* do not demonstrate responses, even to high levels of OS within the first 4 hours and accordingly are likely to respond by a delayed or secondary transcriptional response in the cell. The pattern of early gene regulation by OS is predicted to influence this secondary OS response through alterations in AP-1 family heterodimer formation and ratios resulting from quantitative changes in early phase transcription.

It is recognized that extracellular signals, including OS, have a significant effect upon immediate early gene (IEG) expression. As discussed above, particular care was taken to control for many of these factors including fluctuations in light, temperature and CO₂ content. We show herein that rinsing cells prior to isolating RNA strongly induces the transcription of *FosB, JunB, HO-1,* and other genes *in vitro.* Excluding the rinse step from the protocol eliminates this significant method-induced effect and permits us to isolate and examine the OS-response in the RPE.

Exposing the cells to OS levels of up to 500µM H₂O₂ over 4 hours might be expected to continue to stimulate transcription continuously over the time of exposure. Indeed, we did see an increase *FosB* transcripts over 4-hours. However, with the exception *of FosB,* (and to a minor degree, *JunB*), transcription was stable or declined from the 1- to 4-hour time point for the other genes examined. This suggests that the transcription response is concentration-dependent and not exposure-time dependent. It also may be the case that these genes are simply influenced by a gradient in concentrations, with some genes more responsive than others. For once there were no perceived gradient over the cell a gradient-dependent response would dissipate. These interpretations are also supported by the fact that lower (although not insignificant) levels of OS (up to 100µM H₂O₂) failed to induce the expression of several of the early response genes examined during the 4-hour OS exposure. If the stimulation of transcription was time-dependent, it might be expected that we would see an increase in gene expression over the 4-hour exposure to low levels of OS. Finally, our previous studies comparing IEG expression between a 4-hour OS exposure and 1-hour exposure (followed by our gentle wash method) measured after 4 hours show no differences in levels of induced gene expression.

Three types of early transcription responses to OS were noted in our studies. A dose-dependent response was seen for genes *FosB, CRYBA, FosL,* and *TXN.* A mixed dose-dependent and threshold response was seen for genes *cFos* and *ATF3.* Finally, a threshold response was seen for genes *JunB* and *HO-1.* These results are summarized in Table 2.

**Table 2. Gene-Specific Responses to Oxidative Stress**

| **Dose-Dependent and Mixed OS-Response Genes** | **Threshold OS-Response Genes** | **No Early OS Response** |
|---|---|---|
| *FosB* | *JunB* | *cJun* |
| *cFos* | *HO-1* | *ATF2* |
| *ATF3* | | *CRYBB* |
| *CRYBA* | | *CRYGS* |
| *FosL* | | *GSS* |
| *TXN* | | *CAT* |
| | | *SOD-1* |
| | | *GSR* |
| | | *MSRA* |
| | | *MGST1* |
| | | *TXNIP* |
| | | *NFE2L2* |
| | | *HSP27* |
| | | *IGF- 1* |

These studies demonstrate that different levels of OS can quantitatively regulate the expression of AP-1 transcription factor genes, and other genes modulating the cellular responses to OS. We identified strongly dose-dependent responses in *FosB* and *cFos.* A similar dose-dependent response is seen for the crystallin gene *CRYBA,* but not for three other crystallin genes.

Several genes demonstrate a qualitative threshold response, above which there is a quantitative response to increasing levels of OS. These mixed response genes are members of AP-1 transcription factor families, i.e. *c-Fos* and *ATF3.* For each of these genes, there is no increase in transcription detected below about100 µM H₂O₂; however, above this level of OS, a clearly quantitative response is seen (Table 3).

**Table 3. Fold Changes in Expression in Dose-Dependent and Mixed OS-Response Genes**

| **Oxidative Stress [H₂O₂]** | ***FosB* 1-Hr** | ***FosB* 4-Hr** | ***cFos* 1-Hr** | **c*Fos* 4-Hr** | ***ATF3* 1-Hr.** | ***ATF3* 4-Hr** | ***CRYBA* 1-Hr** | ***CRYBA* 4-Hr** |
|---|---|---|---|---|---|---|---|---|
| 0 µM | 0 | 0 | 0 | 0 | 0 | 0 | 0 | 0 |
| 50 µM | 2 | 2 | 0 | 1 | 0 | 0 | 0 | -2 |
| 100 µM | 2 | 6 | 0 | 2 | 0 | 0 | 1 | 3 |
| 200 µM | 6 | 6 | 4 | 3 | 2 | 3 | 2 | 1 |
| 350 µM | 6 | 20 | 7 | 16 | 2 | 8 | 3 | 3 |
| 500 µM | 16 | 64 | 12 | 32 | 3 | 6 | 4 | 4 |

A threshold response is seen for the gene *HO-1* above about 50 µM H₂O₂. A more quantitative threshold response is seen for *JunB* in which a threshold response was seen for only the highest level of OS at 1-hour. However, a similar level of induced transcription is seen for cells exposed to all levels of OS greater than 50µM H₂O₂ 4-hours after OS (Table 4).

**Table 4. Fold Changes in Threshold Response Genes**

| **Oxidative Stress [H₂O₂]** | ***JunB* 1-Hr** | ***JunB* 4-Hr** | ***HO-1* 1-Hr** | ***HO-1* 4-Hr** |
|---|---|---|---|---|
| 0 µM | 0 | 0 | 0 | 0 |
| 50 µM | 0 | 1 | 2 | 2 |
| 100 µM | 0 | 8 | 2 | 8 |
| 200 µM | 0 | 4 | 2 | 8 |
| 350 µM | 0 | 4 | 2 | 6 |
| 500 µM | 3 | 4 | 2 | 6 |

The selective up-regulation of certain AP-1 family genes *FosB* and *c-Fos* (and to a lesser degree, *FosL*), *JunB* (but not *c-Jun*), and *ATF3* (but not *ATF2*) suggests that the quantitative gene regulation by OS is gene- or promoter-specific and not due simply to a generalized increase in transcription factor gene expression. Without intending to be bound to any particular theory, we suggest that differences in the upstream regulatory elements in *Fos* and *Jun* genes activated by OS, compared to *c-Jun,* may account for the fact that *c-Jun* is not activated by OS. Thus, regulatory elements and transcription factors other than AP-1 may contribute to the pattern of AP-1 gene expression seen following OS.

Another possible mechanism may relate to *c-Jun* regulation during cell growth and proliferation. The confluent status of the cells in our assay may inhibit or down regulate *c-Jun* activation in response to OS. It may be postulated that induction of AP-1 gene expression is due to the role of these transcription factors in regulating the cell cycle. Thus, we may be sampling different stages of activation, independent of the OS. However we do not believe that this is likely, since cells are confluent and undisturbed for three days prior to the OS. Transcription levels for the AP-1 family and other genes in our studies are reproducibly low and consistent. The data suggest that the responses seen are OS-specific.

As discussed, we have also identified three genes that are not transcription factors that show quantitative gene regulation, i.e., *HO-1, CRYBA,* and to a minor degree *TXN. HO-1* and *TXN* are regulated via threshold (*HO-1*) and dose-dependent (*TXN*) signaling, respectively. Seven other OS-response genes were examined and failed to show any up-regulation during the first 4 hours after OS. These genes likely act as secondary responders to OS and may be activated by induction via the AP-1 initial phase response. Quantitative gene regulation of the crystallin *CRYBA* is specific for this chaperone protein and does not appear to reflect non-specific up-regulation of stress-response genes in the cell for the times and conditions investigated. Induced transcription was not seen for three other crystallins, nor for the heat shock protein *HSP27* or transcription factor *NFE2L2.*

### Example 5- Quantitative Assay of Biomarkers for Monitoring OS Levels in a Cell.

The above Examples demonstrate that a stereotypical molecular response to OS exists in cells such as the RPE cells of the eye, and presumably other retinal and non-retinal cell types and tissues. This molecular response, as identified for example in the *FosB* and *JunB* transcription factor genes, appears to be strongly dose-dependent. The dose-dependence can be quantified and used to determine the relative level of stress perceived by a cell under a specific set of environmental conditions.

The studies disclosed herein establish the basis for an inexpensive, high throughput screening methodology that can be used to quantify OS at a cellular level, for example by using quantitative PCR. The method can be physiologically validated through analysis of stress-associated molecular responses in the target RPE, retina, and potentially other tissues. Using this method, it is possible to identify and test antioxidant formulations, minerals and proprietary drugs and formulations thereof at physiologic levels, and to determine their efficacy in reducing the level of OS in the retina and other tissues. Based upon proven efficacy in the target tissue *in vitro,* the cell-based assays offer a useful proxy for, or prerequisite/adjunct to, population-based clinical trials by providing a method to predict the potential therapeutic efficacy of drug formulations in advance of costly human testing.

### Example 6- Antioxidant Screening Matrices

This Example describes an Antioxidant Screening Matrix according to an embodiment of the invention, by which testing of candidate antioxidant molecules and/or compositions can be performed in a high-throughput manner.

### Methods:

RPE or other cell types of interest are grown to confluence in tissue culture wells. A convenient format for many applications is a multi-well plate, such as a 96-well format, depicted schematically in FIGS. 14A and B. Defined culture media is supplemented with varying concentrations of antioxidant vitamins, minerals, or drug formulations according to expected ranges of therapeutic efficacy. Various combinations of these compounds are tested to determine, for example, lowest effective dose, synergistic effects, or other parameters.

To perform the assay, the culture media of test wells is spiked with H₂O₂ (or other oxidizer) to achieve a pre-determined level of OS in the cultures. At one or more selected intervals, for example after 1 hour or 4 hours, RNA is isolated from each well without rinsing, and the level of expression of a cellular marker of stress-induced gene transcription (for example *FosB* or other transcription factor) is determined. As shown above, the level of induction of *FosB* transcription for a given level of OS is predetermined in the system and reflected in values obtained for control cultures subjected to OS without addition of exogenous antioxidant molecules or compositions. Accordingly, any observed changes, either increases or decreases in levels of marker gene induction for a known OS relative to controls, can be attributed to a therapeutic (protective) effect of the antioxidant or other compounds introduced into the tissue culture media.

The antioxidant protection afforded by specific compounds and combinations thereof can be determined and optimized using this method. As will be readily apparent to those of skill in this art, the cell-based assay may be applied not only for the testing on new drugs on a cellular level, but also for the development of proprietary multivitamin or drug formulations.

### Example 7- Antioxidant Modulation of Quantitative Gene Regulation in the Human Retinal Pigment Epithelium (RPE) Under Oxidative Stress (OS)

Examples above describing assays of RPE cells demonstrate quantitative changes in the expression of various genes including transcription factors, chaperone proteins, and antioxidant genes in response to oxidative stress. This example describes an assay in which it is demonstrated that the molecular responses of human RPE cells to measured levels of OS can be modulated by antioxidant vitamin treatment.

### Methods:

Confluent ARPE-19 cells were cultured for three days in defined NR-1 medium in the presence of varying concentrations of vitamin C (0.01 mM to 0.2 mM) to stabilize gene expression. The vitamin C was removed 24 hours prior to treatment with 500 µM H₂O₂. RNA was isolated from the cells using a no-rinse method after 1 hour or 4 hours of OS, and compared to no-OS controls. Gene-specific expression was quantified by real-time PCR on an ABI 7700 System.

### Results:

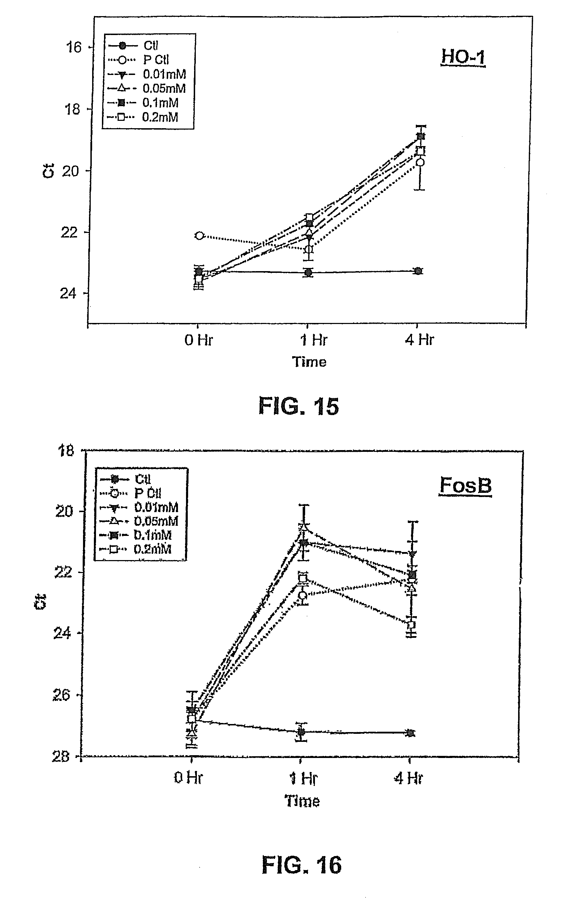

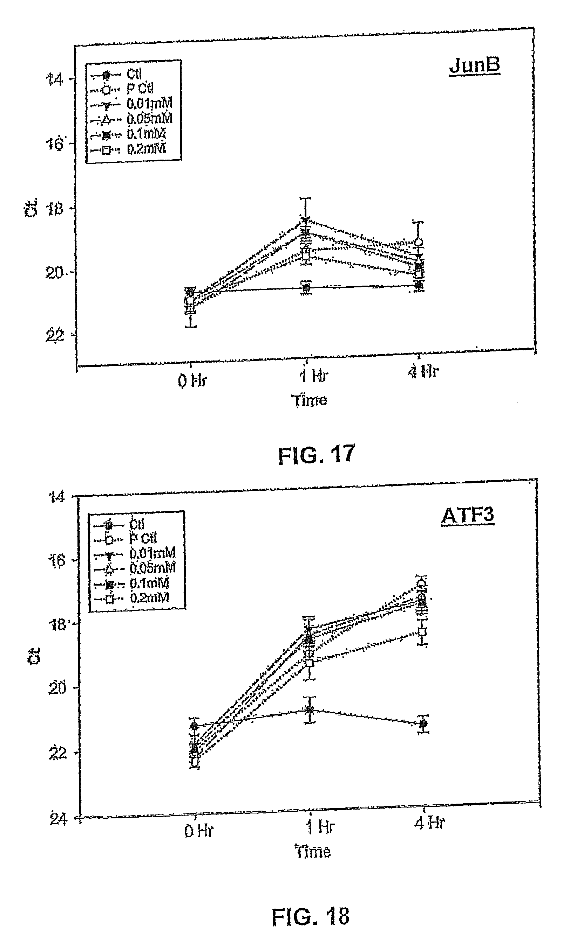

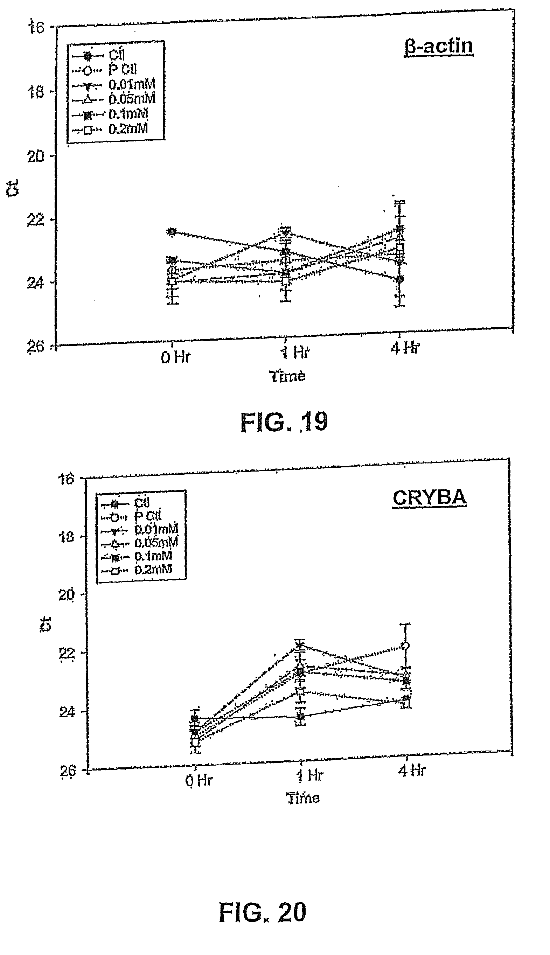

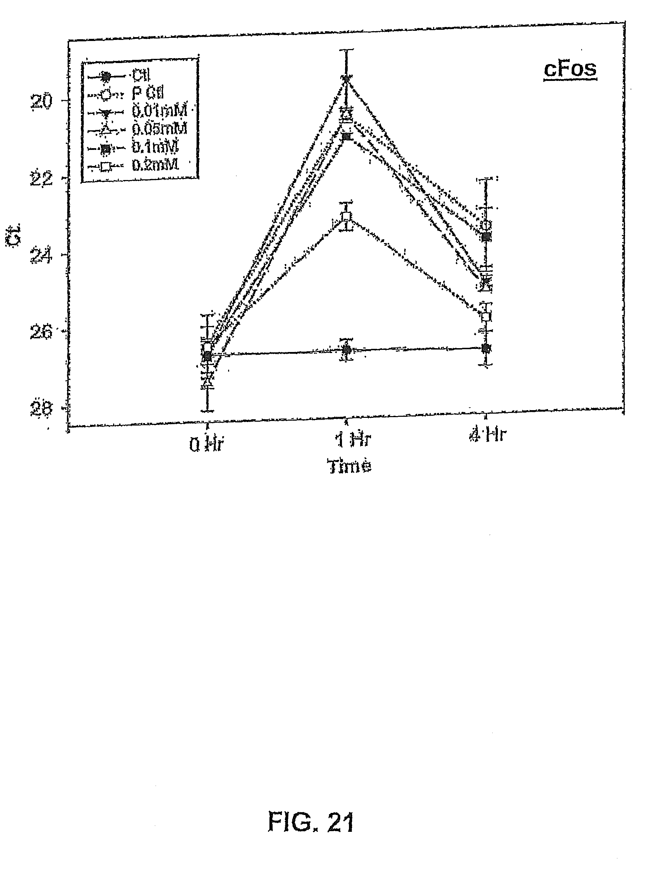

Referring to FIGS. 15-21, expression of four AP-1 transcription factors, crystallin CRYBA and heme oxygenase-1 was quantified. More particularly, expression of the following genes is shown: *HO-1* (FIG. 15); *FosB* (FIG. 16); *JunB* (FIG. 17); *ATF3* (FIG. 18); *CRYBA* (FIG. 20); and *cFOS*(FIG. 21), as well as control gene β-action (FIG. 19).

The data show that quantitative changes in the level of transcription of *FosB, cFos, JunB, ATF3,* and *CRYBA* occurred within one hour of OS. The peak level of induction one hour after OS varies from 5-fold to 128-fold, depending on the gene. Importantly, there is a statistically significant and dose-related reduction in AP-1 gene expression (2- to 8-fold) in cells pretreated with vitamin C. By contrast, heme oxygenase-1 (HO-1) transcription 1 hour and 4 hours after OS does not demonstrate any response to pretreatment with Vitamin C (FIG. 15). The results of this study using an exemplary antioxidant agent, i.e., Vitamin C, demonstrate that treatment with the agent can modulate the expression of AP-1 transcription factors and crystallin *CRYBA* in RPE cells under conditions of oxidative stress. As shown, modulation of the transcriptional response appears to be dose-dependent and quantitative in nature.

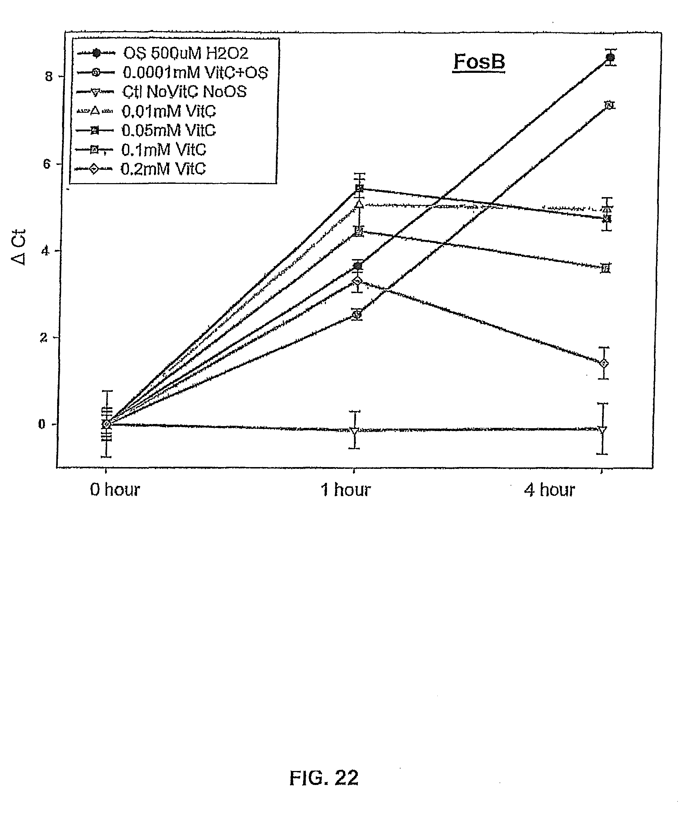

Results of further studies using Vitamin C are shown in FIGS. 22-25. The data show that Vitamin C pretreatment modulates gene expression after oxidative stress (OS) in a preferred panel of genes. Experiments were carried out as described above. Briefly, ARPE19 cells were grown to confluence. Replicate cultures were treated for two days in media containing L-ascorbic acid (Vitamin C) at concentrations of 0.0001, 0.01, 0.05, 0.1 or 0.2 mM, then incubated in medium without Vitamin C for 24 hours, and subjected to oxidative stress (500 µM H₂O₂) for 1 or 4 hours. Following RNA extraction and reverse transcription, real-time PCR was used to assess levels of gene transcripts, as described above. The data presented in FIGS. 22-25 show change in Ct after stress for the different levels of Vitamin C, at an OS of 500 µM H₂O₂ for 1 or 4 hours. The data are normalized to 0 at time 0 in order to minimize the mild variability in starting values for the Ct seen within each experiment. (Ct refers to the cycle at which maximal amplification occurs, and is directly related to the number of gene transcripts present and is a log value.) For example, a change in Ct of 2 is a 4-fold increase in transcription; a change in Ct of 3 is an 8-fold increase, and so on.

The therapeutic effect is demonstrated by a reduction in Ct following OS. OS strongly induces gene expression in our gene panel, and the number of transcripts (amount of induction) is proportional to the level of OS. Therefore, protective effects of an antioxidant such as Vitamin C would be expected to reduce the level of transcripts. A protective effect is reflected in an inverse dose dependent (DD) relationship between the level of antioxidant agent (Vitamin C) and the level of transcripts. Higher pretreatment levels with the antioxidant should lead to a lower level of transcripts after OS (a lower change in Ct). An extremely low dose of Vitamin C (0.0001mM) was chosen in these studies as a homeopathic dose that is not expected to be protective and should be similar to the positive control. This control was added to ensure that all cultures were treated in the same way, as we have determined that even minimal manipulations can have significant effects upon expression of these genes.

The results of these studies are as follows. Figure 22 shows inhibition of OS-induced FosB transcription after pretreatment with Vitamin C. There is a modest dose response at 1 hour, with higher levels of Vitamin C showing slightly reduced levels of *FosB* induction. The positive controls are not transcribed at the level we would predict within 1 hour. However, the effect is quite clear after 4 hours.

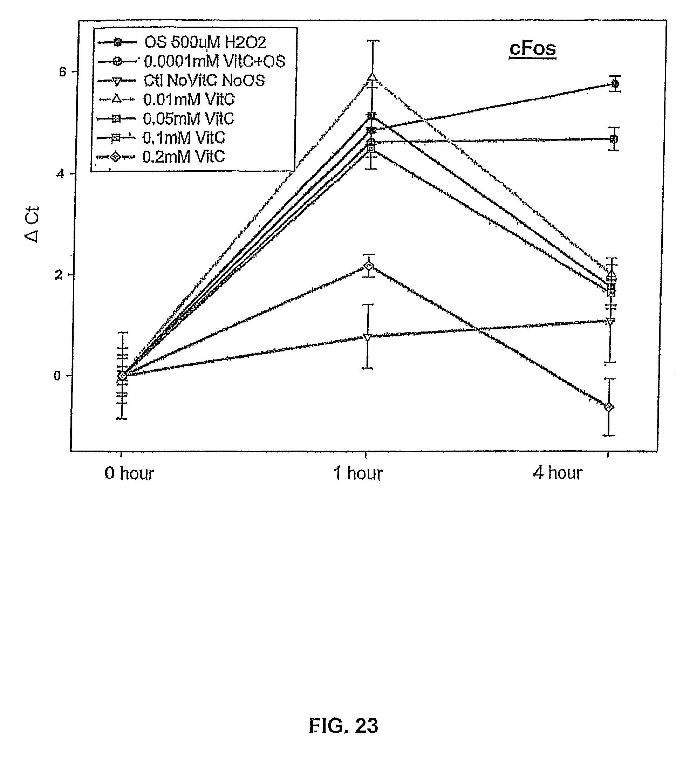

Figure 23 shows corresponding inhibition of OS-induced *cFos* transcription after pretreatment with Vitamin C using a no-rinse method. This gene shows a modest threshold response at 1 hour. There is an apparent dose-dependence at the lower concentrations of Vitamin C. The 0.2mM dose shows a strong dose-dependence at 1 hour. After 4 hours, a threshold response for the lower levels of Vitamin C is still seen.

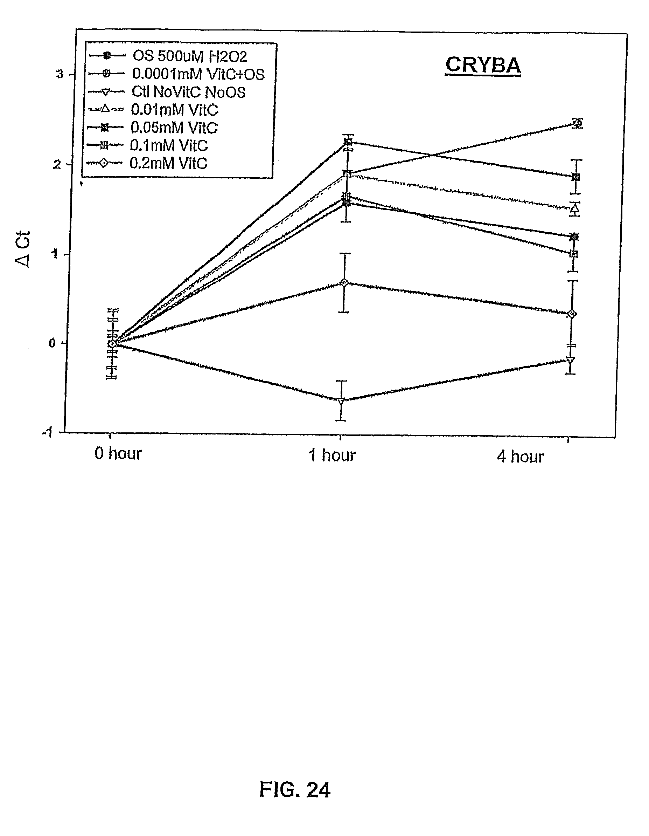

Results for biomarker *CRYBA* are shown in FIG. 24. The DD response is evident at 1 hour, although the positive controls are in the same range. The DD response is more evident at 4 hours.

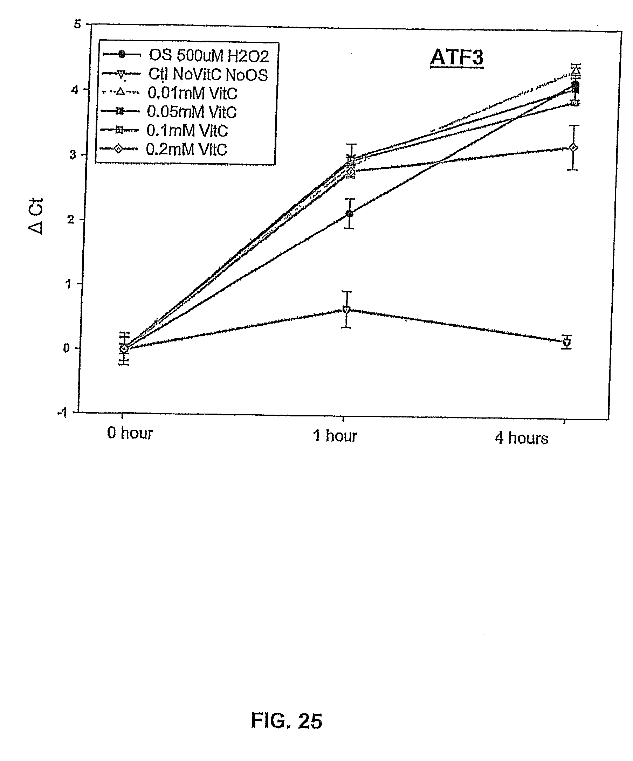

Figure 25 illustrates results for *ATF3.* In the case of this gene, we do not see a response to Vitamin C pretreatment, whereas we do see a strong DD response to OS. A slight threshold response may be present at 4 hours, suggesting that the protective effects of Vitamin C could be gene-specific and not merely due to a non-specific reduction in transcription factor gene expression.

Taken together, results of studies using Vitamin C as a test antioxidant in an RPE cell-based assay system in accordance with the invention show that AP-1 transcription factors *FosB, c-Fos* and *ATF3,* and the crystallin CRYBA are quantitatively up-regulated early in response to oxidative stress in RPE cells. There is a dose-dependent reduction in the activation of transcription of *FosB, c-Fos,* and *CRYBA* by OS following pretreatment with Vitamin C. This effect appears to demonstrate some degree of gene specificity, as the response of *ATF3* and *HO-1* to OS does not appear to be reduced by pretreatment with Vitamin C.

By examining the level of OS-induced transcriptional activation of specific genes such as AP-1 family genes in cell types of choice, one can use the assay to detect and quantify the biological response of cells such as the RPE cell to therapeutic interventions *in vitro.* As demonstrated herein, such molecular-based responses can be used in the assay for assessing the physiological state of the cell and its susceptibility to specific stressors. Furthermore, this Example using a known antioxidant (Vitamin C) demonstrates the utility of the inventive cellular biomarker antioxidant assay to test the efficacy of candidate antioxidant agents for reducing levels of oxidative stress experienced by cells such as the RPE cells of the eye.

### Example 8- Identification of Stress-Induced Biomarkers in RPE Cells

This Example describes methods and results of screening of a gene array to identify genes expressed in human RPE cells that exhibit quantitative changes in mRNA expression in response to oxidative stress. Once identified and confirmed to exhibit a quantitative response to OS, these genes are useful as biomarkers of cellular stress in a cell-based assay according to the invention.

### Materials and Methods:

We performed microarray studies of mRNA from RPE cells exposed to high levels of OS and compared them to control cell mRNA to identify genes that altered transcription in response to OS under experimental conditions described above. Using primer pairs to bracket the probe set regions used on the Affymetrix chip, we performed qPCR analyses of gene expression for a panel of candidate genes that demonstrated a high degree of amplification under OS conditions.

### Results:

This study identified several hundred genes, the expression of which changed more than 2-fold (up or down) in response to oxidative stress. From these, we identified a panel of-25 genes whose expression changed between 4-fold and 50-fold in direct response to the OS (Table 5, *infra*).

**Table 5. Molecular Markers of OS in Cells**

| **Upregulated Gene** | **Activity** | **Fold Change 0 hr vs. Ctrl** | **Fold Change 4hr vs. Ctrl** |
|---|---|---|---|
| C1 Inhibitor | anti-inflammatory neuroprotection? | 64 | 34 |
| AP-1 | transcription factor | | 58 |
| Fos | | 48/34 | 58 |
| Jun B | | 20 | 14 |
| IGFBP's | growth factor binding proteins; independent transcription factors | 3/23 | -5/28 |
| -3 | | | |
| -5 | | 1/4 | 14/3 |
| -6 | | 4 | 4 |
| PLAGL1 (ZAC1/LOT1) | zinc finger transcription factor; apoptosis, cell cycle arrest | 23/4 | 23/8 |
| EGR-1 | early growth response | 20 | 7 |
| TIEG | TGF-β inducible EGR | 10 | 10 |
| P311 | upregulates integrins and GFs | 16/7 | 11/7 |
| Metallothioneins | anti-oxidant enzymes | | |
| 1X | | 7 | 11 |
| 1L | | 7 | 10 |
| 1H | | 5 | 7 |
| 1H-like | | 5 | 8 |
| 1G | | 4 | 6 |
| 2A | | 5 | 8 |
| ETR 101 | immediate early protein | 12 | 11 |
| Thioredoxin δ | anti-oxidant enzyme | 9 | 9 |
| Heat shock protein | chaperone | | |
| HSP A1A | | 4 | 8 |
| HSP A1B | | 4 | 4 |
| Interleukin 8 | pro-inflammatory cytokine | 3 | 45 |
| M-GST3 | anti-oxidant enzyme | 3 | 4 |
| GSTA4 | anti-oxidant enzyme | 2 | 3 |
| MMP 2 | matrix metalloproteinase | 9 | 11 |
| DTR | growth factor receptor | 2/4 | 9/12 |

The panel of candidate genes included genes from several functional classes including: antioxidant proteins; transcription factors (TF); anti-apoptotic proteins; and chaperone proteins. Temporal changes in the transcription of some of these genes were also seen at different time points after induction of OS.

### REFERENCES

It is believed that a review of the references will increase appreciation of the present invention. The disclosures of all references cited herein are incorporated herein by reference.
Age-Related Eye Disease Study Research Group. The Age-Related Eye Disease Study (AREDS): design implications. AREDS Report No. 1. Control Clin Trials. 1999 Dec;20(6):573-600.
Age-Related Eye Disease Study Research Group. Risk factors associated with age-related macular degeneration. A case-control study in the age-related eye disease study: Age-Related Eye Disease Study Report No. 3. Ophthalmology. 2000 Dec;107(12):2224-32.
Age-Related Eye Disease Study Research Group. The age-related eye disease study (AREDS) system for classifying cataracts from photographs: AREDS Report No. 4. Am J Ophthalmol. 2001 1 Feb;131 (2):167-75.
Age-Related Eye Disease Study Research Group. Risk factors associated with age-related nuclear and cortical cataract: a case-control study in the Age-Related Eye Disease Study, AREDS Report No. 5. Ophthalmology. 2001 Aug;108(8):1400-8.
Age-Related Eye Disease Study Research Group. The effect of five-year zinc supplementation on serum zinc, serum cholesterol and hematocrit in persons randomly assigned to treatment group in the age-related eye disease study: AREDS Report No. 7. J Nutr. 2002 Apr;132(4):697-702
Age-Related Eye Disease Study Research Group. A randomized, placebo-controlled, clinical trial of high-dose supplementation with vitamins C and E, beta carotene, and zinc for age-related macular degeneration and vision loss: AREDS Report No. 8. Arch Ophthalmol. 2001 Oct;119(10):1417-36.
Age-Related Eye Disease Study Research Group. A randomized, placebo-controlled, clinical trial of high-dose supplementation with vitamins C and E and beta carotene for age-related cataract and vision loss: AREDS Report No. 9. Arch Ophthalmol. 2001 Oct;119(10):1439-52.
Seigel D. AREDS investigators distort findings. Arch Ophthalmol. 2002 Jan;120(1):100-1.
Sackett CS, Schenning S. The age-related eye disease study: the results of the clinical trial. Insight. 2002 Jan-Mar;27(1):5-7.
Hammond BR Jr, Johnson MA. The age-related eye disease study (AREDS) Nutr Rev. 2002 Sep;60(9):283-8.

The invention has been described in detail with reference to preferred embodiments thereof. However, it will be appreciated that those skilled in the art, upon consideration of this disclosure, may make modifications and improvements within the spirit and scope of the invention.

## Claims

1. A method for measuring oxidative stress in a cell, comprising:
(a) providing isolated cell populations, each population comprising a cell type that expresses at least one biomarker of oxidative stress (OS) that responds to OS by changing its expression level in a quantitative manner, said cells maintained under conditions in which said expression level is unchanged in absence of an inducer of OS;
(b) providing an inducer of OS to a test group and not to a control group of the cell populations of step (a), and
(c) determining the level of expression of said biomarker in the test and control groups of step (b), wherein a change in the expression level of said biomarker in the test group is correlated with the level of oxidative stress in the cell.

2. The method of claim 1, wherein the biomarker of OS is selected from the group consisting *of FosB, JunB, cFos, Fos L, ATF3, CRYBA, TXN,* heme oxygenase (*HO-1*), *EGR-1*, Cl inhibitor, *AP-1, IGFBP-3, IGFBP-5*, IGFBP-6, PLAGLI* (*ZAC1*/*LOT1*), *TIEG, P311,* metallothionein 1X, metallothionein 1L, metallothionein 1H, metallothionein 1H-like, metallothionein 1G, metallothionein 2A, *ETR 101,* thioredoxin δ3 *HSPA1A, HSPA1B, HSP-27,* interleukin 8, *M-GST3, GSTA4, MMP2, DTR, HOS-1,* and *LEDGF.*

3. The method of claim 1, wherein the level of expression of the biomarker changes in response to the level of OS in a dose-dependent manner.

4. The method of claim 3, wherein the biomarker of OS is selected from the group consisting of *FosB, Jun B, cFos, ATF3,* and *CRYBA.*

5. The method of claim 1, wherein measurement of the level of expression of the biomarker of OS in a cell or population is determined from mRNA or protein.

6. The method of claim 5, wherein mRNA level is determined using polymerase chain reaction (PCR).

7. The method of claim 1, wherein the cell population comprises a cell type of the retina.

8. The method of claim 7, wherein the cell type is a retinal pigmented epithelial (RPE) cell.
